# EUROPEAN PATENT APPLICATION

(11) **EP 2 659 906 A1**
(43) Date of publication of application: **06.11.2013**
(21) Application number: 12166314.0
(22) Date of filing: 01.05.2012
(51) Int. Cl.: A61K 39/00

(54) **Compositions**

(71) Applicant: Affiris AG, 1030 Wien (AT)
(72) Inventor: Mandler, Markus, 1100 Vienna (AT); Zauner, Wolfgang, 1030 Vienna (AT); Mattner, Frank, 1190 Vienna (AT); Schmidt, Walter, 1020 Vienna (AT)
(74) Representative: Sonn & Partner Patentanwälte

(57) **Abstract**

The present invention relates to a composition comprising at least one mimotope of an epitope of alpha-synuclein for use in a method for preventing and/or treating ß-amyloidoses including Alzheimer's disease, wherein said at least one mimotope is coupled or fused to a pharmaceutically acceptable carrier protein selected from the group consisting of a non-toxic diphtheria toxin mutant, keyhole limpet hemocyanin (KLH), diphtheria toxin (DT), tetanus toxid (TT) and Haemophilus influenzae protein D (protein D).

## Description

The present invention relates to the prevention and treatment of diseases associated with ß-amyloid formation and/or aggregation (ß-Amyloidoses).

Various degenerative diseases are characterized by the accumulation and polymerization of misfolded specific proteins. These so called proteopathies include disorders such as Alzheimer's disease (AD), Parkinson's disease (PD) and Huntington's disease (HD) or inclusion body myositis (IBM) as well as systemic entities including various amyloidoses.

The present invention relates to the prevention, treatment and diagnosis of AD associated with the accumulation and aggregation of misfolded protein alpha Synuclein (a-syn). Other examples of diseases targeted by this invention include but are not limited to Fronto-temporal dementia (FTD), progressive supranuclear palsy (PSP) as well as Dementia in Down syndrome (DS) and IBM.

a-syn (initially identified as PARK1 and PARK4) is a 140 amino acid protein widely expressed in the human nervous system including brain areas such as neocortex, hippocampus, dentate gyrus, olfactory bulb, striatum, thalamus and cerebellum. In the nervous system it is predominantly found in the pre-synaptic termini and although its role is not completely understood it has been associated with normal synaptic function. a-syn is also highly expressed in members of the hematopoietic lineage including B-, T-, and NK cells as well as monocytes and platelets. While its exact role in all of these cells is not known to date, it has been demonstrated to be involved in the differentiation of megakaryocytes (platelet precursors).

As shown previously, a-syn is an important component of the amyloidogenic inclusions found in neurons and glia present in the brains of patients with PD and multiple system atrophy (MSA), respectively. These inclusions represent the typical pathological alterations of these prominent synucleinopathies. This along with other evidence implies aggregated misfoldeda-syn as being the agent ultimately causing these disorders.

Importantly, inclusions of this misfolded protein have also been identified in several other degenerative disorders including AD, FTD, PSP, DS and IBM.

In a transgenic mouse model for Dementia with Lewy Bodies (DLB) it has been recently shown that co-expression of human a-syn and human APP leads to the development of cognitive- and motoric alterations associated with the loss of cholinergic neurons, the reduction in synaptic vesicles, formation of extensive amyloid plaques, and a-syn-immunoreactive intra-neuronal fibrillar inclusions. The phenotype in double transgenic mice was much more severe as compared to single transgenic animals indicating a synergistic effect of the coexpression of the two molecules.

Although the exact mechanisms by which accumulation of a-syn functionally impairs and finally leads to the demise of neurons are not fully understood, recent studies imply that accumulation of abnormally folded a-syn is involved in the degenerative processes underlying the above mentioned proteopathies.

In Iwatsubo T. (Neuropathology 27 (5)(2007): 474-478) the correlation of alpha-synuclein depositions as well as its phosphorylation with a pathogenesis of alpha-synucleopathies is examined. The author of this publication found that serine 129 of alpha-synuclein deposited in synucleopathy lesions is extensively phosphorylated. US 2007/213253 relates to mutant human alpha-synuclein as well as peptides derived therefrom which may be used for inhibiting the aggregation of the wild-type human alpha-synuclein. In the WO 2004/041067 means and methods for preventing or treating diseases associated with alpha-synuclein aggregation are disclosed which comprise the use of alpha-synuclein fragments. In the US 2003/166558 peptides are described which can be used to induce immune response to protein deposits. US 2005/198694 relates to alpha-synuclein fragments comprising at least 100 amino acids and having a C-terminal deletion of 1 to 23 amino acids.

It is an object of the present invention to provide compounds and medicaments which can be used to treat and/or prevent Alzheimer's disease.

The present invention relates to a composition comprising at least one mimotope of an epitope of alpha-synuclein for use in a method for preventing and/or treating ß-amyloidoses including Alzheimer's disease, wherein said at least one mimotope is coupled or fused, preferably coupled, to a pharmaceutically acceptable carrier protein selected from the group consisting of a non-toxic diphtheria toxin mutant, keyhole limpet hemocyanin (KLH), diphtheria toxin (DT), tetanus toxid (TT) and Haemophilus influenzae protein D (protein D).

It surprisingly turned out that mimotopes of an epitope of alpha-synuclein can be used to treat diseases which are associated with beta-amyloid deposits in brains.

Even though AD is generally considered a proteopathy driven by extensive deposits of amyloid beta (Aß) and hyperphosphotylated Tau, abnormal aggregation and accumulation of the synaptic protein a-syn might be associated with plaque formation in AD. Interestingly, a-syn was originally identified as a component of the amyloid-enriched fraction from AD patient-brain, underlining the potential importance of a-syn for AD (Uéda K. et al. Proc. Natl. Acad. Sci. U.S.A. 90 (23) 1993: 11282-6; A. Iwai, T. Saitoh et al. Neuron, 14 (1995), pp. 467-475). In addition, Matsubara et al. (Dement Geriatr Cogn Disord 2001;12:106-109 ) also identified an association between AD and certain variants of the a-syn gene in humans.

Regarding the mechanism(s) by which a-syn and for example Aß interact pathophysiologically in the aforementioned disease, it has been postulated that they could directly interact by engaging synergistic neurodegenerative pathways. It has been recently shown that pathologically folded Aß- as well as a-syn molecules can mutually exacerbate their toxic effects in preclinical model systems of human diseases (Masliah et al. PNAS 2001 vol. 98, no. 21 p.12245-12250). Obviously, these findings provide a molecular basis and, thus, indicate a critical role for Aß, a-syn and in particular their cooperation in different neurodegenerative conditions.

Hence, reduction of a-syn accumulation and oligomerisation shows to be be beneficial with regard to the treatment of diseases associated with misfolded a-syn, especially of AD, FTD, PSP, DS and IBM and, thus, presents a novel strategy for causal treatment of these degenerative diseases exceeding the mere alleviation of symptoms resulting from current treatment strategies.

The immunogenicity of the mimotopes can surprisingly be increased if the mimotopes are fused or coupled to a carrier protein selected from the group consisting of a non-toxic diphtheria toxin mutant, keyhole limpet hemocyanin (KLH), diphtheria toxin (DT), tetanus toxid (TT) and Haemophilus influenzae protein D (protein D), whereby non-toxic diphtheria toxin mutants, such as CRM197, are particularly preferred.

As used herein, the term "epitope" refers to an immunogenic region of an antigen which is recognized by a particular antibody molecule. An antigen may possess one or more epitopes, each capable of binding an antibody that recognizes the particular epitope.

According to the present invention the term "mimotope" refers to a molecule which has a conformation that has a topology equivalent to the epitope of which it is a mimic. The mimotope binds to the same antigen-binding region of an antibody which binds immunospecifically to a desired antigen. The mimotope will elicit an immunological response in a host that is reactive to the antigen to which it is a mimic. The mimotope may also act as a competitor for the epitope of which it is a mimic in in vitro inhibition assays (e.g. ELISA inhibition assays) which involve the epitope and an antibody binding to said epitope. However, a mimotope of the present invention may not necessarily prevent or compete with the binding of the epitope of which it is a mimic in an in vitro inhibition assay although it is capable to induce a specific immune response when administered to a mammal. The compounds of the present invention comprising such mimotopes (also those listed above) have the advantage to avoid the formation of autoreactive T-cells, since the peptides of the compounds have an amino acid sequence which varies from those of naturally occurring amyloid-beta peptide.

The mimotopes of the present invention can be synthetically produced by chemical synthesis methods which are well known in the art, either as an isolated peptide or as a part of another peptide or polypeptide. Alternatively, the peptide mimotope can be produced in a microorganism which produces the peptide mimotope which is then isolated and if desired, further purified. The peptide mimotope can be produced in microorganisms such as bacteria, yeast or fungi, in eukaryote cells such as a mammalian or an insect cell, or in a recombinant virus vector such as adenovirus, poxvirus, herpesvirus, Simliki forest virus, baculovirus, bacteriophage, sindbis virus or sendai virus. Suitable bacteria for producing the peptide mimotope include E.coli, B.subtilis or any other bacterium that is capable of expressing peptides such as the peptide mimotope. Suitable yeast types for expressing the peptide mimotope include Saccharomyces cerevisiae, Schizosaccharomyces pombe, Candida, Pichia pastoris or any other yeast capable of expressing peptides. Corresponding methods are well known in the art. Also methods for isolating and purifying recombinantly produced peptides are well known in the art and include e.g. as gel filtration, affinity chromatography, ion exchange chromatography etc.

To facilitate isolation of the peptide mimotope, a fusion polypeptide may be made wherein the peptide mimotope is translationally fused (covalently linked) to a heterologous polypeptide which enables isolation by affinity chromatography. Typical heterologous polypeptides are His-Tag (e.g. His₆; 6 histidine residues), GST-Tag (Glutathione-S-transferase) etc.. The fusion polypeptide facilitates not only the purification of the mimotopes but can also prevent the mimotope polypeptide from being degraded during purification. If it is desired to remove the heterologous polypeptide after purification the fusion polypeptide may comprise a cleavage site at the junction between the peptide mimotope and the heterologous polypeptide. The cleavage site consists of an amino acid sequence that is cleaved with an enzyme specific for the amino acid sequence at the site (e.g. proteases).

The mimotopes of the present invention may also be modified at or nearby their N- and/or C-termini so that at said positions a cysteine residue is bound thereto.

The mimotopes according to the present invention preferably are antigenic polypeptides which in their amino acid sequence vary from the amino acid sequence of alpha synuclein. In this respect, the inventive mimotopes may not only comprise amino acid substitutions of one or more naturally occurring amino acid residues but also of one or more non-natural amino acids (i.e. not from the 20 "classical" amino acids) or they may be completely assembled of such non-natural amino acids. Suitable antibody-inducing antigens may be provided from commercially available peptide libraries. Preferably, these peptides are at least 7 amino acids, and preferred lengths may be up to 16, preferably up to 14 or 20 amino acids (e.g. 5 to 16 amino acid residues). According to the invention, however, also longer peptides may very well be employed as antibody-inducing antigens. Furthermore the mimotopes of the present invention may also be part of a polypeptide and consequently comprising at their N- and/or C-terminus at least one further amino acid residue.

For preparing the mimotopes of the present invention (i.e. the antibody-inducing antigens disclosed herein), of course also phage libraries, peptide libraries are suitable, for instance produced by means of combinatorial chemistry or obtained by means of high throughput screening techniques for the most varying structures (Display: A Laboratory Manual by Carlos F. Barbas (Editor), et al.; Willats WG Phage display: practicalities and prospects. Plant Mol. Biol. 2002 Dec.; 50(6):837-54).

As used herein, the term "epitope" refers to an immunogenic region of an antigen to which a particular antibody molecule can specifically bind thereto. An antigen may possess one or more epitopes, each capable of binding an antibody that recognizes the particular epitope.

The composition of the present invention may comprise at least one, at least 2, at least 3, at least 4, at least 5 or at least 10 mimotopes as defined herein.

According to a preferred embodiment of the present invention the non-toxic diphtheria toxin mutant is selected from the group consisting of CRM 197, CRM 176, CRM 228, CRM 45, CRM 9, CRM 102, CRM 103 and CRM 107, whereby CRM 197 is particularly preferred.

The mimotopes of the present invention are particularly preferred fused or conjugated to non-toxic diphtheria toxin mutants, such as CRM 197 (a nontoxic but antigenically identical variant of diphtheria toxin), CRM 176, CRM 228, CRM 45 (Uchida et al J. Biol. Chem. 218; 3838-3844, 1973), CRM 9, CRM 45, CRM 102, CRM 103 and CRM 107 and other mutations described by Nicholls and Youle in Genetically Engineered Toxins, Ed: Frankel, Marcel Dekker Inc, 1992). Methods for fusing peptides like mimotopes to other peptides, polypeptides or proteins are well known in the art.

Another aspect of the present invention relates to a composition comprising at least one mimotope of an epitope of alpha-synuclein for use in a method for preventing and/or treating ß-amyloidoses including Alzheimer's disease

In such a composition the at least one mimotope can be fused or conjugated to a pharmaceutically acceptable carrier, preferably KLH (Keyhole Limpet Hemocyanin), tetanus toxoid, albumin-binding protein, bovine serum albumin, a dendrimer (MAP; Biol. Chem. 358: 581), peptide linkers (or flanking regions) as well as the substances described in Singh et al., Nat. Biotech. 17 (1999), 1075-1081 (in particular those in Table 1 of that document), and O'Hagan et al., Nature Reviews, Drug Discovery 2 (9) (2003), 727-735 (in particular the endogenous immuno-potentiating compounds and delivery systems described therein), or mixtures thereof. The conjugation chemistry (e.g. via heterobifunctional compounds such as GMBS and of course also others as described in "Bioconjugate Techniques", Greg T. Hermanson) in this context can be selected from reactions known to the skilled man in the art. Of course the at least one mimotope can also be fused or conjugated to a pharmaceutically acceptable carrier protein selected from the group consisting of a non-toxic diphtheria toxin mutant, keyhole limpet hemocyanin (KLH), diphtheria toxin (DT), tetanus toxid (TT) and Haemophilus influenzae protein D (protein D) as defined above.

The composition of the present invention may be administered by any suitable mode of application, e.g. i.d., i.v., i.p., i.m., intranasally, orally, subcutaneously, transdermally, intradermally and in any suitable delivery device (O'Hagan et al., Nature Reviews, Drug Discovery 2 (9), (2003), 727-735). Therefore, that at least one mimotope of the present invention is preferably formulated for intravenous, subcutaneous, intradermal or intramuscular administration (see e.g. "Handbook of Pharmaceutical Manufacturing Formulations", Sarfaraz Niazi, CRC Press Inc, 2004).

The composition according to the present invention comprises the mimotope according to the invention in an amount of from 0.1 ng to 10 mg, preferably 10 ng to 1 mg, in particular 100 ng to 100 µg, or, alternatively, e.g. 100 fmol to 10 µmol, preferably 10 pmol to 1 µmol, in particular 100 pmol to 100 nmol. Typically, the vaccine may also contain auxiliary substances, e.g. buffers, stabilizers etc.

Typically, the composition of the present invention may also comprise auxiliary substances, e.g. buffers, stabilizers etc.. Preferably, such auxiliary substances, e.g. a pharmaceutically acceptable excipient, such as water, buffer and/or stabilisers, are contained in an amount of 0.1 to 99 %(weight), more preferred 5 to 80%(weight), especially 10 to 70 %(weight). Possible administration regimes include a weekly, biweekly, fourweekly (monthly) or bimonthly treatment for about 1 to 12 months; however, also 2 to 5, especially 3 to 4, initial vaccine administrations (in one or two months), followed by boaster vaccinations 6 to 12 months thereafter or even years thereafter are preferred - besides other regimes already suggested for other vaccines.

According to a preferred embodiment of the present invention the at least one mimotope is administered to an individual in an amount of 0.1 ng to 10 mg, preferably of 0.5 to 500 µg, more preferably 1 to 100 µg, per immunization. In a preferred embodiment these amounts refer to all mimotopes present in the composition of the present invention. In another preferred embodiment these amounts refer to each single mimotopes present in the composition. It is of course possible to provide a vaccine in which the various mimotopes are present in different or equal amounts. However, the mimotopes of the present invention may alternatively be administered to an individual in an amount of 0.1 ng to 10 mg, preferably 10 ng to 1 mg, in particular 100 ng to 300 µg/kg body weight.

The amount of mimotopes that may be combined with the carrier materials to produce a single dosage form will vary depending upon the host treated and the particular mode of administration. The dose of the composition may vary according to factors such as the disease state, age, sex and weight of the individual, and the ability of antibody to elicit a desired response in the individual. Dosage regime may be adjusted to provide the optimum therapeutic response. For example, several divided doses may be administered daily or the dose may be proportionally reduced as indicated by the exigencies of the therapeutic situation. The dose of the vaccine may also be varied to provide optimum preventative dose response depending upon the circumstances. For instance, the mimotopes and compositions of the present invention may be administered to an individual at intervals of several days, one or two weeks or even months or years depending always on the level of antibodies induced by the administration of the composition of the present invention.

In a preferred embodiment of the present invention the composition is applied between 2 and 10, preferably between 2 and 7, even more preferably up to 5 and most preferably up to 4 times. This number of immunizations may lead to a basic immunisation. In a particularly preferred embodiment the time interval between the subsequent vaccinations is chosen to be between 2 weeks and 5 years, preferably between 1 month and up to 3 years, more preferably between 2 months and 1.5 years. An exemplified vaccination schedule may comprise 3 to 4 initial vaccinations over a period of 6 to 8 weeks and up to 6 months. Thereafter the vaccination may be repeated every two to ten years. The repeated administration of the mimotopes of the present invention may maximize the final effect of a therapeutic vaccination.

According to a preferred embodiment of the present invention the at least one mimotope is formulated with at least one adjuvant.

"Adjuvants" are compounds or a mixture that enhance the immune response to an antigen (i.e. mimotope). Antigens may act primarily as a delivery system, primarily as an immune modulator or have strong features of both. Suitable adjuvants include those suitable for use in mammals, including humans.

According to a particular preferred embodiment of the present invention the at least one adjuvant used in the composition as defined herein is capable to stimulate the innate immune system.

Innate immune responses are mediated by toll-like receptors (TLR's) at cell surfaces and by Nod-LRR proteins (NLR) intracellularly and are mediated by D1 and D0 regions respectively. The innate immune response includes cytokine production in response to TLR activation and activation of Caspase-1 and IL-1β secretion in response to certain NLRs (including Ipaf). This response is independent of specific antigens, but can act as an adjuvant to an adaptive immune response that is antigen specific. The antigen may be supplied externally in the form of a vaccine or infection, or may be indigenous, for example, as is the case for tumor-associated antigens.

A number of different TLRs have been characterized. These TLRs bind and become activated by different ligands, which in turn are located on different organisms or structures. The development of immunopotentiator compounds that are capable of eliciting responses in specific TLRs is of interest in the art. For example, US 4,666,886 describes certain lipopeptide molecules that are TLR2 agonists. WO 2009/118296, WO 2008/005555, WO 2009/111337 and WO 2009/067081 each describe classes of small molecule agonists of TLR7. WO 2007/040840 and WO 2010/014913 describe TLR7 and TLR8 agonists for treatment of diseases. These various compounds include small molecule immunopotentiators (SMIPs).

The at least one adjuvant capable to stimulate the innate immune system preferably comprises or consists of a Toll-like receptor (TLR) agonist, preferably a TLR1, TLR2, TLR3, TLR4, TLR5, TLR7, TLR8 or TLR9 agonist, particularly preferred a TLR4 agonist.

Agonists of Toll-like receptors are well known in the art. For instance a TLR 2 agonist is Pam3CysSerLys4, peptidoglycan (Ppg), PamCys, a TLR3 agonist is IPH 31XX, a TLR4 agonist is an Aminoalkyl glucosaminide phosphate, E6020, CRX-527, CRX-601, CRX-675, 5D24.D4, RC-527, a TLR7 agonist is Imiquimod, 3M-003, Aldara, 852A, R850, R848, CL097, a TLR8 agonist is 3M-002, a TLR9 agonist is Flagellin, Vaxlmmune, CpG ODN (AVE0675, HYB2093), CYT005-15 AllQbG10, dSLIM.

According to a preferred embodiment of the present invention the TLR agonist is selected from the group consisting of monophosphoryl lipid A (MPL), 3-de-O-acylated monophosphoryl lipid A (3D-MPL), poly I:C, GLA, flagellin, R848, imiquimod and CpG.

The composition of the present invention may comprise MPL. MPL may be synthetically produced MPL or MPL obtainable from natural sources. Of course it is also possible to add to the composition of the present invention chemically modified MPL. Examples of such MPL's are known in the art.

According to a further preferred embodiment of the present invention the at least one adjuvant comprises or consists of a saponin, preferably QS21, a water in oil emulsion and a liposome.

The at least one adjuvant is preferably selected from the group consisting of MF59, AS01, AS02, AS03, AS04, aluminium hydroxide and aluminium phosphate.

Examples of known suitable delivery-system type adjuvants that can be used in humans include, but are not limited to, alum (e.g., aluminum phosphate, aluminum sulfate or aluminum hydroxide), calcium phosphate, liposomes, oil-in-water emulsions such as MF59 (4.3% w/v squalene, 0.5% w/v polysorbate 80 (Tween 80), 0.5% w/v sorbitan trioleate (Span 85)), water-in-oil emulsions such as Montanide, and poly(D,L-lactide-co-glycolide) (PLG) microparticles or nanoparticles.

Examples of known suitable immune modulatory type adjuvants that can be used in humans include, but are not limited to saponins extracts from the bark of the Aquilla tree (QS21, Quil A), TLR4 agonists such as MPL (Monophosphoryl Lipid A), 3DMPL (3-O-deacylated MPL) or GLA-AQ, LT/CT mutants, cytokines such as the various interleukins (e.g., IL-2, IL-12) or GM-CSF, and the like.

Examples of known suitable immune modulatory type adjuvants with both delivery and immune modulatory features that can be used in humans include, but are not limited to ISCOMS (see, e.g., Sjölander et al. (1998) J. Leukocyte Biol. 64:713; WO90/03184, WO96/11711 WO 00/48630, WO98/36772, WO00/41720, WO06/134423 and WO07/026,190) or GLA-EM which is a combination of a Toll-like receptor agonists such as a TLR4 agonist and an oil-in-water emulsion.

Further exemplary adjuvants to enhance effectiveness of the mimotope compositions of the present invention include, but are not limited to: (1) oil-in-water emulsion formulations (with or without other specific immunostimulating agents such as muramyl peptides (see below) or bacterial cell wall components), such as for example (a) SAF, containing 10% Squalane, 0.4% Tween 80, 5% pluronic-blocked polymer L121, and thr-MDP either microfluidized into a submicron emulsion or vortexed to generate a larger particle size emulsion, and (b) RIBI™ adjuvant system (RAS), (Ribi Immunochem, Hamilton, Mont.) containing 2% Squalene, 0.2% Tween 80, and one or more bacterial cell wall components such as mono-phosphorylipid A (MPL), trehalose dimycolate (TDM), and cell wall skeleton (CWS), preferably MPL+CWS (DETOX™); (2) saponin adjuvants, such as QS21, STIMULON™ (Cambridge Bioscience, Worcester, Mass.), Abisco® (Isconova, Sweden), or Iscomatrix® (Commonwealth Serum Laboratories, Australia), may be used or particles generated therefrom such as ISCOMs (immunostimulating complexes), which ISCOMS may be devoid of additional detergent e.g. WO00/07621; (3) Complete Freund's Adjuvant (CFA) and Incomplete Freund's Adjuvant (IFA); (4) cytokines, such as interleukins (e.g. IL-1, IL-2, IL-4, IL-5, IL-6, IL-7, IL-12 (WO99/44636), etc.), interferons (e.g. gamma interferon), macrophage colony stimulating factor (M-CSF), tumor necrosis factor (TNF), etc.; (5) monophosphoryl lipid A (MPL) or 3-O-deacylated MPL (3dMPL) (see e.g., GB-2220221, EP-A-0689454), optionally in the substantial absence of alum when used with pneumococcal saccharides (see e.g. WO00/56358); (6) combinations of 3dMPL with, for example, QS21 and/or oil-in-water emulsions (see e.g. EP-A-0835318, EP-A-0735898, EP-A-0761231); (7) a polyoxyethylene ether or a polyoxyethylene ester (see e.g. WO99/52549); (8) a polyoxyethylene sorbitan ester surfactant in combination with an octoxynol (WO01/21207 or a polyoxyethylene alkyl ether or ester surfactant in combination with at least one additional non-ionic surfactant such as an octoxynol (WO01/21152); (9) a saponin and an immunostimulatory oligonucleotide (e.g. a CpG oligonucleotide) (WO00/62800); (10) an immunostimulant and a particle of metal salt (see e.g. WO00/23105); (11) a saponin and an oil-in-water emulsion e.g. WO99/11241 (12) a saponin (e.g. QS21)+3dMPL+IM2 (optionally+a sterol) e.g. WO98/57659; (13) other substances that act as immunostimulating agents to enhance the efficacy of the composition. Muramyl peptides include N-acetyl-muramyl-L-threonyl-D-isoglutamine (thr-MDP), N-25 acetylnormnuramyl-L-alanyl-D-isoglutamine (nor-MDP), N-acetylmuramyl-L-alanyl-D-isoglutaminyl-L-alanine-2-(1'-2'-dipalmitoyl-sn-glycero-3-hydroxyphosphoryloxy)-ethylamine MTP-PE), etc.

Particularly preferred compositions of the present invention comprise as adjuvant an oil-in-water emulsion with or without Toll-like receptor agonists, as well as liposomes and/or saponin-containing adjuvants, with or without Toll-like receptor agonists. The composition of the present invention may also comprise aluminium hydroxide with or without Toll-like receptor agonists as adjuvant.

According to a preferred embodiment of the present invention the epitope comprises or consists of the amino acid sequence KNEEGAP or DMPVDPDN.

Mimotopes of the aforementioned epitopes are known to the person skilled in the art (see e.g. WO 2009/103105, WO 2011/020133).

The composition according to the present invention comprises preferably at least one mimotope comprising or consisting of the amino acid sequence

(X₁)ₙX₂X₃X₄X₅GX₆P(X₇)ₘ (Formula I),

wherein
X₁ is any amino acid residue,
X₂ is an amino acid residue selected from the group consisting of lysine (K), arginine (R), alanine (A) and histidine
(H),
X₃ is an amino acid residue selected from the group consisting of asparagine (N), glutamine (Q), serine (S), glycine (G) and alanine (A), preferably asparagine (N), serine (S), glycine (G) and alanine (A),
X₄ is an amino acid residue selected from the group consisting of glutamic acid (E), aspartic acid (D) and alanine (A), X₅ is an amino acid residue selected from the group consisting of glutamic acid (E) and aspartic acid (D), X₆ is an amino acid residue selected from the group consisting of alanine (A) and tyrosine (Y),
X₇ is any amino acid residue,
n and m, independently, are 0 or an integer of more than 0,
wherein the amino acid sequence according to Formula I is not identical with, or does not comprise the 7-mer polypeptide fragment of alpha-synuclein having the amino acid sequence KNEEGAP, and wherein
the at least one mimotope comprising the amino acid sequence according to Formula I has a binding capacity to an antibody which is specific for an epitope of alpha-synuclein comprising the amino acid sequence KNEEGAP.

The term "peptide having a binding capacity to an antibody which is specific for an epitope of alpha-synuclein" means that said peptide can be bound to alpha-synuclein specific antibody which has been produced by the administration of alpha-synuclein or fragments thereof to a mammal. Said peptide having said binding capacity is able to induce the formation of alpha-synuclein specific antibodies in a mammal. The latter antibodies bind consequently to the compound of the present invention as well as to alpha-synuclein.

According to a particularly preferred embodiment of the present invention X₂ is an amino acid residue selected from the group consisting of lysine (K) and arginine (R) and/or X₆ is alanine (A).

According to a preferred embodiment of the present invention the mimotope comprises or consists of an amino acid sequence selected from the group consisting of (X₁)ₙKNDEGAP(X₇)ₘ, (X₁)ₙANEEGAP(X₇)ₘ, (X₁)ₙKAEEGAP(X₇)ₘ, (X₁)ₙKNAEGAP(X₇)ₘ, (X₁)ₙRNEEGAP(X₇)ₘ, (X₁)ₙHNEEGAP(X₇)ₘ, (X₁)ₙKNEDGAP(X₇)ₘ, (X₁)ₙKQEEGAP(X₇)ₘ, (X₁)ₙKSEEGAP(X₇)ₘ, (X₁)ₙKNDDGAP(X₇)ₘ, (X₁)ₙRNDEGAP(X₇)ₘ, (X₁)ₙRNEDGAP(X₇)ₘ, (X₁)ₙRQEEGAP(X₇)ₘ, (X₁)ₙRSEEGAP(X₇)ₘ, (X₁)ₙANDEGAP(X₇)ₘ, (X₁)ₙANEDGAP(X₇)ₘ, (X₁)ₙHSEEGAP(X₇)ₘ, (X₁)ₙASEEGAP(X₇)ₘ, (X₁)ₙHNEDGAP(X₇)ₘ, (X₁)ₙHNDEGAP(X₇)ₘ, (X₁)ₙRNAEGAP(X₇)ₘ, (X₁)ₙHNAEGAP(X₇)ₘ, (X₁)ₙKSAEGAP(X₇)ₘ, (X₁)ₙKSDEGAP(X₇)ₘ, (X₁)ₙKSEDGAP(X₇)ₘ, (X₁)ₙRQDEGAP(X₇)ₘ, (X₁)ₙRQEDGAP(X₇)ₘ, (X₁)ₙHSAEGAP(X₇)ₘ, (X₁)ₙRSAEGAP(X₇)ₘ, (X₁)ₙRSDEGAP(X₇)ₘ, (X₁)ₙRSEDGAP(X₇)ₘ, (X₁)ₙHSDEGAP(X₇)ₘ, (X₁)ₙHSEDGAP(X₇)ₘ, (X₁)ₙRQDDGAP(X₇)ₘ, preferably (X₁)ₙKNDEGAP(X₂)m, (X₁)ₙRNEEGAP(X₂)m, (X₁)ₙRNDEGAP(X₂)m, (X₁)ₙKNAEGAP(X₂)m, (X₁)ₙKSDEGAP(X₂)ₘ, (X₁)ₙRNAEGAP(X₂)m, Or (X₁)ₙRSEEGAP(X₂)m.

The composition according to the present invention comprises preferably at least one mimotope comprising or consisting of an amino acid sequence selected from the group consisting of (X₁)ₙQASFAME (X₇)ₘ, (X₁)ₙTASWKGE(X₇)ₘ, (X₁)ₙQASSKLD (X₇)ₘ, (X₁)ₙTPAWKGE(X₇)ₘ, (X₁)ₙTPSWAGE(X₇)ₘ, (X₁)ₙTPSWKGE(X₇)ₘ,
wherein
X₁ is any amino acid residue,
X₇ is any amino acid residue,
n and m, independently, are 0 or an integer of more than 0,
said at least one mimotope having a binding capacity to an antibody which is specific for an epitope of alpha-synuclein comprising the amino acid sequence KNEEGAP
for use in preventing and/or treating synucleinopathies.

According to a preferred embodiment of the present invention the at least one mimotope comprises the amino acid sequence

(X_{1'})_{n'}X_{2'}X_{3'}PVX_{4'}X_{5'}X_{6'}(X_{7'})_{m'} (Formula II),

wherein
X_{1'} is any amino acid residue,
X_{2'} is an amino acid residue selected from the group consisting of aspartic acid (D) and glutamic acid (E), X_{3'} is any amino acid residue,
X_{4'} is any amino acid residue,
X_{5'} is an amino acid residue selected from the group consisting of proline (P) and alanine (A),
X_{6'} is an amino acid residue selected from the group consisting of aspartic acid (D) and glutamic acid (E), X_{7'} is any amino acid residue,
n' and m', independently, are 0 or an integer of more than 0,
wherein the amino acid sequence according to Formula II is not identical with, or does not comprise the 8-mer polypeptide fragment of alpha-synuclein having the amino acid sequence DMPVDPDN, and wherein
the at least one mimotope comprising the amino acid sequence according to Formula II has a binding capacity to an antibody which is specific for an epitope of alpha-synuclein comprising the amino acid sequence DMPVDPDN.

According to a preferred embodiment of the present invention X_{3'} is an amino acid residue selected from the group consisting of glutamine (Q), serine (S), threonine (T), arginine (R), asparagine (N), valine (V), histidine (H), methionine (M), tyrosine (Y), alanine (A) and leucin (L).

According to a particularly preferred embodiment of the present invention X_{4'} is an amino acid residue selected from the group consisting of glutamine (Q), tryptophane (W), threonine (T), arginine (R), aspartic acid(D), isoleucin (I), valine (V), histidine (H), proline (P), tyrosine (Y), alanine (A), serine (S) and leucin (L).

The mimotope of the present invention which is part of the composition of the present invention has preferably an amino acid sequence selected from the group consisting of (C)DQPVLPD, (C)DMPVLPD, (C)DSPVLPD, (C)DSPVWAE, (C)DTPVLAE, (C)DQPVLPDN, (C)DMPVLPDN, (C)DSPVLPDN, (C)DQPVTAEN, (C)DSPVWAEN, (C)DTPVLAEN, (C)HDRPVTPD, (C)DRPVTPD, (C)DVPVLPD, (C)DTPVYPD, (C)DTPVIPD, (C)HDRPVTPDN, (C)DRPVTPDN, (C)DNPVHPEN, (C)DVPVLPDN, (C)DTPVYPDN, (C)DTPVIPDN, (C)DQPVLPDG, (C)DMPVLPDG, (C)DSPVLPDG, (C)DSPVWAEG, (C)DRPVAPEG, (C)DHPVHPDS, (C)DMPVSPDR, (C)DSPVPPDD, (C)DQPVYPDI, (C)DRPVYPDI, (C)DHPVTPDR, (C)EYPVYPES, (C)DTPVLPDS, (C)DMPVTPDT, (C)DAPVTPDT, (C)DSPVVPDN, (C)DLPVTPDR, (C)DSPVHPDT, (C)DAPVRPDS, (C)DMPVWPDG, (C)DAPVYPDG, (C)DRPVQPDR, (C)YDRPVQPDR, (C)DMPVDPEN, (C)DMPVDADN, DQPVLPD(C), DMPVLPD(C), (C)EMPVDPDN and (C)DNPVHPE.

According to a particularly preferred embodiment of the present invention n' and/or m' are 1 and X_{1'} and/or X_{7'} are cysteine (C).

According to a preferred embodiment of the present invention the mimotope comprises 7 to 30, preferably 7 to 20, more preferably 7 to 16, most preferably 8 or 9, amino acid residues.

According to a preferred embodiment of the present invention the mimotope comprises or consists of an amino acid sequence selected from the group consisting of DQPVLPD, DSPVLPD, DVPVLPD, DSPVLPDG, YDRPVQPDR, DHPVHPDS, DAPVRPDS, KNDEGAP, KQEEGAP and KSEEGAP, in particular DQPVLPD and YDRPVQPDR. Of course, in order to facilitate coupling of these mimotopes to a carrier protein as defined herein, the mimotopes may comprise at the C- and/or N-terminal end a cysteine residue

According to a particularly preferred embodiment of the present invention the composition of the present invention comprises the following combinations of mimotopes and carriers and/or adjuvants (see Table A).

### Table A:

Mimotope sequences (SEQ): A = DQPVLPD, B = DSPVLPD, C = DVPVLPD, D = DSPVLPDG, E = YDRPVQPDR, F = DHPVHPDS, G = DAPVRPDS, H = KNDEGAP, I = KQEEGAP, J = KSEEGAP (the mimotopes comprise either a C- or N-terminal cysteine residue for coupling them to the carrier molecule)

Carrier (CAR): C1 = CRM197, C2 = KLH

Adjuvant (ADJ): A1 = Alum, A2 = saponin based formulation, A3 = QS21 (pure), A4 = squalene based formulation, A5 = Addavax (Sorbitan trioleate (0.5% w/v) in squalene oil (5% v/v) - Tween 80 (0.5% w/v) in sodium citrate buffer (10 mM, pH 6.5)), A6 = MF59 (0.5% Polysorbate 80, 0.5% Sorbitan Triolate, 4.3% Squalene, water for injection, 10 mM Na-citrate buffer), A7 = AS03, A8 = AF03, A9 = monophosphoryl-lipid A (MPL), A10 = MPLA (derivative of lipid A from Salmonella minnesota lipopolysaccharide), A11 = synthetic MPL, A12 = A1+A3, A13 = A1+A5, A14 = A1+A9, A15 = A3+A9, A16 = A3+A4, A17 = A4+A9, A18 = A3+A4+A9, A19 = A1+A3+A4, A20 = A1+A4+A9, A21 = A1+A3+A9, A22 = Ribi adjuvant system, A23 = QS21 (encapsulated), A24 = CpG, A25 = A1+A23, A26 = A1+A24, A27 = A1+A2, A28 = A1+A9+A24, A29 = A1+A3+A24, A30 = A1+A23+A24, A31 = A4+A3, A32 = A4+A9, A33 = A4+A23, A34 = A4+A24, A35 = A4+A9+A24, A36 = A4+A3+A24, A37 = A4+A23+A24, A38 = A4+A3+A9, A39 = A4+A23+A9, A40 = A4+A3+A9+A24, A41 = A4+A23+A9+A24, A42 = A9+A23, A43 = A1+A3+A9+A24, A44 = A1+A9+A23+A24

| **No.** | **SEQ** | **CAR** | **ADJ** | **No.** | **SEQ** | **CAR** | **ADJ** | **No.** | **SEQ** | **CAR** | **ADJ** |
|---|---|---|---|---|---|---|---|---|---|---|---|
| **1** | A | C1 | A1 | **294** | D | C1 | A30 | **587** | G | C2 | A15 |
| **2** | B | C1 | A1 | **295** | E | C1 | A30 | **588** | H | C2 | A15 |
| **3** | C | C1 | A1 | **296** | F | C1 | A30 | **589** | I | C2 | A15 |
| **4** | D | C1 | A1 | **297** | G | C1 | A30 | **590** | J | C2 | A15 |
| **5** | E | C1 | A1 | **298** | H | C1 | A30 | **591** | A | C2 | A16 |
| **6** | F | C1 | A1 | **299** | I | C1 | A30 | **592** | B | C2 | A16 |
| **7** | G | C1 | A1 | **300** | J | C1 | A30 | **593** | C | C2 | A16 |
| **8** | H | C1 | A1 | **301** | A | C1 | A31 | **594** | D | C2 | A16 |
| **9** | I | C1 | A1 | **302** | B | C1 | A31 | **595** | E | C2 | A16 |
| **10** | J | C1 | A1 | **303** | C | C1 | A31 | **596** | F | C2 | A16 |
| **11** | A | C1 | A2 | **304** | D | C1 | A31 | **597** | G | C2 | A16 |
| **12** | B | C1 | A2 | **305** | E | C1 | A31 | **598** | H | C2 | A16 |
| **13** | C | C1 | A2 | **306** | F | C1 | A31 | **599** | I | C2 | A16 |
| **14** | D | C1 | A2 | **307** | G | C1 | A31 | **600** | J | C2 | A16 |
| **15** | E | C1 | A2 | **308** | H | C1 | A31 | **601** | A | C2 | A17 |
| **16** | F | C1 | A2 | **309** | I | C1 | A31 | **602** | B | C2 | A17 |
| **17** | G | C1 | A2 | **310** | J | C1 | A31 | **603** | C | C2 | A17 |
| **18** | H | C1 | A2 | **311** | A | C1 | A32 | **604** | D | C2 | A17 |
| **19** | I | C1 | A2 | **312** | B | C1 | A32 | **605** | E | C2 | A17 |
| **20** | J | C1 | A2 | **313** | C | C1 | A32 | **606** | F | C2 | A17 |
| **21** | A | C1 | A3 | **314** | D | C1 | A32 | **607** | G | C2 | A17 |
| **22** | B | C1 | A3 | **315** | E | C1 | A32 | **608** | H | C2 | A17 |
| **23** | C | C1 | A3 | **316** | F | C1 | A32 | **609** | I | C2 | A17 |
| **24** | D | C1 | A3 | **317** | G | C1 | A32 | **610** | J | C2 | A17 |
| **25** | E | C1 | A3 | **318** | H | C1 | A32 | **611** | A | C2 | A18 |
| **26** | F | C1 | A3 | **319** | I | C1 | A32 | **612** | B | C2 | A18 |
| **27** | G | C1 | A3 | **320** | J | C1 | A32 | **613** | C | C2 | A18 |
| **28** | H | C1 | A3 | **321** | A | C1 | A33 | **614** | D | C2 | A18 |
| **29** | I | C1 | A3 | **322** | B | C1 | A33 | **615** | E | C2 | A18 |
| **30** | J | C1 | A3 | **323** | C | C1 | A33 | **616** | F | C2 | A18 |
| **31** | A | C1 | A4 | **324** | D | C1 | A33 | **617** | G | C2 | A18 |
| **32** | B | C1 | A4 | **325** | E | C1 | A33 | **618** | H | C2 | A18 |
| **33** | C | C1 | A4 | **326** | F | C1 | A33 | **619** | I | C2 | A18 |
| **34** | D | C1 | A4 | **327** | G | C1 | A33 | **620** | J | C2 | A18 |
| **35** | E | C1 | A4 | **328** | H | C1 | A33 | **621** | A | C2 | A19 |
| **36** | F | C1 | A4 | **329** | I | C1 | A33 | **622** | B | C2 | A19 |
| **37** | G | C1 | A4 | **330** | J | C1 | A33 | **623** | C | C2 | A19 |
| **38** | H | C1 | A4 | **331** | A | C1 | A34 | **624** | D | C2 | A19 |
| **39** | I | C1 | A4 | **332** | B | C1 | A34 | **625** | E | C2 | A19 |
| **40** | J | C1 | A4 | **333** | C | C1 | A34 | **626** | F | C2 | A19 |
| **41** | A | C1 | A5 | **334** | D | C1 | A34 | **627** | G | C2 | A19 |
| **42** | B | C1 | A5 | **335** | E | C1 | A34 | **628** | H | C2 | A19 |
| **43** | C | C1 | A5 | **336** | F | C1 | A34 | **629** | I | C2 | A19 |
| **44** | D | C1 | A5 | **337** | G | C1 | A34 | **630** | J | C2 | A19 |
| **45** | E | C1 | A5 | **338** | H | C1 | A34 | **631** | A | C2 | A20 |
| **46** | F | C1 | A5 | **339** | I | C1 | A34 | **632** | B | C2 | A20 |
| **47** | G | C1 | A5 | **340** | J | C1 | A34 | **633** | C | C2 | A20 |
| **48** | H | C1 | A5 | **341** | A | C1 | A35 | **634** | D | C2 | A20 |
| **49** | I | C1 | A5 | **342** | B | C1 | A35 | **635** | E | C2 | A20 |
| **50** | J | C1 | A5 | **343** | C | C1 | A35 | **636** | F | C2 | A20 |
| **51** | A | C1 | A6 | **344** | D | C1 | A35 | **637** | G | C2 | A20 |
| **52** | B | C1 | A6 | **345** | E | C1 | A35 | **638** | H | C2 | A20 |
| **53** | C | C1 | A6 | **346** | F | C1 | A35 | **639** | I | C2 | A20 |
| **54** | D | C1 | A6 | **347** | G | C1 | A35 | **640** | J | C2 | A20 |
| **55** | E | C1 | A6 | **348** | H | C1 | A35 | **641** | A | C2 | A21 |
| **56** | F | C1 | A6 | **349** | I | C1 | A35 | **642** | B | C2 | A21 |
| **57** | G | C1 | A6 | **350** | J | C1 | A35 | **643** | C | C2 | A21 |
| **58** | H | C1 | A6 | **351** | A | C1 | A36 | **644** | D | C2 | A21 |
| **59** | I | C1 | A6 | **352** | B | C1 | A36 | **645** | E | C2 | A21 |
| **60** | J | C1 | A6 | **353** | C | C1 | A36 | **646** | F | C2 | A21 |
| **61** | A | C1 | A7 | **354** | D | C1 | A36 | **647** | G | C2 | A21 |
| **62** | B | C1 | A7 | **355** | E | C1 | A36 | **648** | H | C2 | A21 |
| **63** | C | C1 | A7 | **356** | F | C1 | A36 | **649** | I | C2 | A21 |
| **64** | D | C1 | A7 | **357** | G | C1 | A36 | **650** | J | C2 | A21 |
| **65** | E | C1 | A7 | **358** | H | C1 | A36 | **651** | A | C2 | A22 |
| **66** | F | C1 | A7 | **359** | I | C1 | A36 | **652** | B | C2 | A22 |
| **67** | G | C1 | A7 | **360** | J | C1 | A36 | **653** | C | C2 | A22 |
| **68** | H | C1 | A7 | **361** | A | C1 | A37 | **654** | D | C2 | A22 |
| **69** | I | C1 | A7 | **362** | B | C1 | A37 | **655** | E | C2 | A22 |
| **70** | J | C1 | A7 | **363** | C | C1 | A37 | **656** | F | C2 | A22 |
| **71** | A | C1 | A8 | **364** | D | C1 | A37 | **657** | G | C2 | A22 |
| **72** | B | C1 | A8 | **365** | E | C1 | A37 | **658** | H | C2 | A22 |
| **73** | C | C1 | A8 | **366** | F | C1 | A37 | **659** | I | C2 | A22 |
| **74** | D | C1 | A8 | **367** | G | C1 | A37 | **660** | J | C2 | A22 |
| **75** | E | C1 | A8 | **368** | H | C1 | A37 | **661** | A | C2 | A23 |
| **76** | F | C1 | A8 | **369** | I | C1 | A37 | **662** | B | C2 | A23 |
| **77** | G | C1 | A8 | **370** | J | C1 | A37 | **663** | C | C2 | A23 |
| **78** | H | C1 | A8 | **371** | A | C1 | A38 | **664** | D | C2 | A23 |
| **79** | I | C1 | A8 | **372** | B | C1 | A38 | **665** | E | C2 | A23 |
| **80** | J | C1 | A8 | **373** | C | C1 | A38 | **666** | F | C2 | A23 |
| **81** | A | C1 | A9 | **374** | D | C1 | A38 | **667** | G | C2 | A23 |
| **82** | B | C1 | A9 | **375** | E | C1 | A38 | **668** | H | C2 | A23 |
| **83** | C | C1 | A9 | **376** | F | C1 | A38 | **669** | I | C2 | A23 |
| **84** | D | C1 | A9 | **377** | G | C1 | A38 | **670** | J | C2 | A23 |
| **85** | E | C1 | A9 | **378** | H | C1 | A38 | **671** | A | C2 | A24 |
| **86** | F | C1 | A9 | **379** | I | C1 | A38 | **672** | B | C2 | A24 |
| **87** | G | C1 | A9 | **380** | J | C1 | A38 | **673** | C | C2 | A24 |
| **88** | H | C1 | A9 | **381** | A | C1 | A39 | **674** | D | C2 | A24 |
| **89** | I | C1 | A9 | **382** | B | C1 | A39 | **675** | E | C2 | A24 |
| **90** | J | C1 | A9 | **383** | C | C1 | A39 | **676** | F | C2 | A24 |
| **91** | A | C1 | A10 | **384** | D | C1 | A39 | **677** | G | C2 | A24 |
| **92** | B | C1 | A10 | **385** | E | C1 | A39 | **678** | H | C2 | A24 |
| **93** | C | C1 | A10 | **386** | F | C1 | A39 | **679** | I | C2 | A24 |
| **94** | D | C1 | A10 | **387** | G | C1 | A39 | **680** | J | C2 | A24 |
| **95** | E | C1 | A10 | **388** | H | C1 | A39 | **681** | A | C2 | A25 |
| **96** | F | C1 | A10 | **389** | I | C1 | A39 | **682** | B | C2 | A25 |
| **97** | G | C1 | A10 | **390** | J | C1 | A39 | **683** | C | C2 | A25 |
| **98** | H | C1 | A10 | **391** | A | C1 | A40 | **684** | D | C2 | A25 |
| **99** | I | C1 | A10 | **392** | B | C1 | A40 | **685** | E | C2 | A25 |
| **100** | J | C1 | A10 | **393** | C | C1 | A40 | **686** | F | C2 | A25 |
| **101** | A | C1 | A11 | **394** | D | C1 | A40 | **687** | G | C2 | A25 |
| **102** | B | C1 | A11 | **395** | E | C1 | A40 | **688** | H | C2 | A25 |
| **103** | C | C1 | A11 | **396** | F | C1 | A40 | **689** | I | C2 | A25 |
| **104** | D | C1 | A11 | **397** | G | C1 | A40 | **690** | J | C2 | A25 |
| **105** | E | C1 | A11 | **398** | H | C1 | A40 | **691** | A | C2 | A26 |
| **106** | F | C1 | A11 | **399** | I | C1 | A40 | **692** | B | C2 | A26 |
| **107** | G | C1 | A11 | **400** | J | C1 | A40 | **693** | C | C2 | A26 |
| **108** | H | C1 | A11 | **401** | A | C1 | A41 | **694** | D | C2 | A26 |
| **109** | I | C1 | A11 | **402** | B | C1 | A41 | **695** | E | C2 | A26 |
| **110** | J | C1 | A11 | **403** | C | C1 | A41 | **696** | F | C2 | A26 |
| **111** | A | C1 | A12 | **404** | D | C1 | A41 | **697** | G | C2 | A26 |
| **112** | B | C1 | A12 | **405** | E | C1 | A41 | **698** | H | C2 | A26 |
| **113** | C | C1 | A12 | **406** | F | C1 | A41 | **699** | I | C2 | A26 |
| **114** | D | C1 | A12 | **407** | G | C1 | A41 | **700** | J | C2 | A26 |
| **115** | E | C1 | A12 | **408** | H | C1 | A41 | **701** | A | C2 | A27 |
| **116** | F | C1 | A12 | **409** | I | C1 | A41 | **702** | B | C2 | A27 |
| **117** | G | C1 | A12 | **410** | J | C1 | A41 | **703** | C | C2 | A27 |
| **118** | H | C1 | A12 | **411** | A | C1 | A42 | **704** | D | C2 | A27 |
| **119** | I | C1 | A12 | **412** | B | C1 | A42 | **705** | E | C2 | A27 |
| **120** | J | C1 | A12 | **413** | C | C1 | A42 | **706** | F | C2 | A27 |
| **121** | A | C1 | A13 | **414** | D | C1 | A42 | **707** | G | C2 | A27 |
| **122** | B | C1 | A13 | **415** | E | C1 | A42 | **708** | H | C2 | A27 |
| **123** | C | C1 | A13 | **416** | F | C1 | A42 | **709** | I | C2 | A27 |
| **124** | D | C1 | A13 | **417** | G | C1 | A42 | **710** | J | C2 | A27 |
| **125** | E | C1 | A13 | **418** | H | C1 | A42 | **711** | A | C2 | A28 |
| **126** | F | C1 | A13 | **419** | I | C1 | A42 | **712** | B | C2 | A28 |
| **127** | G | C1 | A13 | **420** | J | C1 | A42 | **713** | C | C2 | A28 |
| **128** | H | C1 | A13 | **421** | A | C1 | A43 | **714** | D | C2 | A28 |
| **129** | I | C1 | A13 | **422** | B | C1 | A43 | **715** | E | C2 | A28 |
| **130** | J | C1 | A13 | **423** | C | C1 | A43 | **716** | F | C2 | A28 |
| **131** | A | C1 | A14 | **424** | D | C1 | A43 | **717** | G | C2 | A28 |
| **132** | B | C1 | A14 | **425** | E | C1 | A43 | **718** | H | C2 | A28 |
| **133** | C | C1 | A14 | **426** | F | C1 | A43 | **719** | I | C2 | A28 |
| **134** | D | C1 | A14 | **427** | G | C1 | A43 | **720** | J | C2 | A28 |
| **135** | E | C1 | A14 | **428** | H | C1 | A43 | **721** | A | C2 | A29 |
| **136** | F | C1 | A14 | **429** | I | C1 | A43 | **722** | B | C2 | A29 |
| **137** | G | C1 | A14 | **430** | J | C1 | A43 | **723** | C | C2 | A29 |
| **138** | H | C1 | A14 | **431** | A | C1 | A44 | **724** | D | C2 | A29 |
| **139** | I | C1 | A14 | **432** | B | C1 | A44 | **725** | E | C2 | A29 |
| **140** | J | C1 | A14 | **433** | C | C1 | A44 | **726** | F | C2 | A29 |
| **141** | A | C1 | A15 | **434** | D | C1 | A44 | **727** | G | C2 | A29 |
| **142** | B | C1 | A15 | **435** | E | C1 | A44 | **728** | H | C2 | A29 |
| **143** | C | C1 | A15 | **436** | F | C1 | A44 | **729** | I | C2 | A29 |
| **144** | D | C1 | A15 | **437** | G | C1 | A44 | **730** | J | C2 | A29 |
| **145** | E | C1 | A15 | **438** | H | C1 | A44 | **731** | A | C2 | A30 |
| **146** | F | C1 | A15 | **439** | I | C1 | A44 | **732** | B | C2 | A30 |
| **147** | G | C1 | A15 | **440** | J | C1 | A44 | **733** | C | C2 | A30 |
| **148** | H | C1 | A15 | **441** | A | C2 | A1 | **734** | D | C2 | A30 |
| **149** | I | C1 | A15 | **442** | B | C2 | A1 | **735** | E | C2 | A30 |
| **150** | J | C1 | A15 | **443** | C | C2 | A1 | **736** | F | C2 | A30 |
| **151** | A | C1 | A16 | **444** | D | C2 | A1 | **737** | G | C2 | A30 |
| **152** | B | C1 | A16 | **445** | E | C2 | A1 | **738** | H | C2 | A30 |
| **153** | C | C1 | A16 | **446** | F | C2 | A1 | **739** | I | C2 | A30 |
| **154** | D | C1 | A16 | **447** | G | C2 | A1 | **740** | J | C2 | A30 |
| **155** | E | C1 | A16 | **448** | H | C2 | A1 | **741** | A | C2 | A31 |
| **156** | F | C1 | A16 | **449** | I | C2 | A1 | **742** | B | C2 | A31 |
| **157** | G | C1 | A16 | **450** | J | C2 | A1 | **743** | C | C2 | A31 |
| **158** | H | C1 | A16 | **451** | A | C2 | A2 | **744** | D | C2 | A31 |
| **159** | I | C1 | A16 | **452** | B | C2 | A2 | **745** | E | C2 | A31 |
| **160** | J | C1 | A16 | **453** | C | C2 | A2 | **746** | F | C2 | A31 |
| **161** | A | C1 | A17 | **454** | D | C2 | A2 | **747** | G | C2 | A31 |
| **162** | B | C1 | A17 | **455** | E | C2 | A2 | **748** | H | C2 | A31 |
| **163** | C | C1 | A17 | **456** | F | C2 | A2 | **749** | I | C2 | A31 |
| **164** | D | C1 | A17 | **457** | G | C2 | A2 | **750** | J | C2 | A31 |
| **165** | E | C1 | A17 | **458** | H | C2 | A2 | **751** | A | C2 | A32 |
| **166** | F | C1 | A17 | **459** | I | C2 | A2 | **752** | B | C2 | A32 |
| **167** | G | C1 | A17 | **460** | J | C2 | A2 | **753** | C | C2 | A32 |
| **168** | H | C1 | A17 | **461** | A | C2 | A3 | **754** | D | C2 | A32 |
| **169** | I | C1 | A17 | **462** | B | C2 | A3 | **755** | E | C2 | A32 |
| **170** | J | C1 | A17 | **463** | C | C2 | A3 | **756** | F | C2 | A32 |
| **171** | A | C1 | A18 | **464** | D | C2 | A3 | **757** | G | C2 | A32 |
| **172** | B | C1 | A18 | **465** | E | C2 | A3 | **758** | H | C2 | A32 |
| **173** | C | C1 | A18 | **466** | F | C2 | A3 | **759** | I | C2 | A32 |
| **174** | D | C1 | A18 | **467** | G | C2 | A3 | **760** | J | C2 | A32 |
| **175** | E | C1 | A18 | **468** | H | C2 | A3 | **761** | A | C2 | A33 |
| **176** | F | C1 | A18 | **469** | I | C2 | A3 | **762** | B | C2 | A33 |
| **177** | G | C1 | A18 | **470** | J | C2 | A3 | **763** | C | C2 | A33 |
| **178** | H | C1 | A18 | **471** | A | C2 | A4 | **764** | D | C2 | A33 |
| **179** | I | C1 | A18 | **472** | B | C2 | A4 | **765** | E | C2 | A33 |
| **180** | J | C1 | A18 | **473** | C | C2 | A4 | **766** | F | C2 | A33 |
| **181** | A | C1 | A19 | **474** | D | C2 | A4 | **767** | G | C2 | A33 |
| **182** | B | C1 | A19 | **475** | E | C2 | A4 | **768** | H | C2 | A33 |
| **183** | C | C1 | A19 | **476** | F | C2 | A4 | **769** | I | C2 | A33 |
| **184** | D | C1 | A19 | **477** | G | C2 | A4 | **770** | J | C2 | A33 |
| **185** | E | C1 | A19 | **478** | H | C2 | A4 | **771** | A | C2 | A34 |
| **186** | F | C1 | A19 | **479** | I | C2 | A4 | **772** | B | C2 | A34 |
| **187** | G | C1 | A19 | **480** | J | C2 | A4 | **773** | C | C2 | A34 |
| **188** | H | C1 | A19 | **481** | A | C2 | A5 | **774** | D | C2 | A34 |
| **189** | I | C1 | A19 | **482** | B | C2 | A5 | **775** | E | C2 | A34 |
| **190** | J | C1 | A19 | **483** | C | C2 | A5 | **776** | F | C2 | A34 |
| **191** | A | C1 | A20 | **484** | D | C2 | A5 | **777** | G | C2 | A34 |
| **192** | B | C1 | A20 | **485** | E | C2 | A5 | **778** | H | C2 | A34 |
| **193** | C | C1 | A20 | **486** | F | C2 | A5 | **779** | I | C2 | A34 |
| **194** | D | C1 | A20 | **487** | G | C2 | A5 | **780** | J | C2 | A34 |
| **195** | E | C1 | A20 | **488** | H | C2 | A5 | **781** | A | C2 | A35 |
| **196** | F | C1 | A20 | **489** | I | C2 | A5 | **782** | B | C2 | A35 |
| **197** | G | C1 | A20 | **490** | J | C2 | A5 | **783** | C | C2 | A35 |
| **198** | H | C1 | A20 | **491** | A | C2 | A6 | **784** | D | C2 | A35 |
| **199** | I | C1 | A20 | **492** | B | C2 | A6 | **785** | E | C2 | A35 |
| **200** | J | C1 | A20 | **493** | C | C2 | A6 | **786** | F | C2 | A35 |
| **201** | A | C1 | A21 | **494** | D | C2 | A6 | **787** | G | C2 | A35 |
| **202** | B | C1 | A21 | **495** | E | C2 | A6 | **788** | H | C2 | A35 |
| **203** | C | C1 | A21 | **496** | F | C2 | A6 | **789** | I | C2 | A35 |
| **204** | D | C1 | A21 | **497** | G | C2 | A6 | **790** | J | C2 | A35 |
| **205** | E | C1 | A21 | **498** | H | C2 | A6 | **791** | A | C2 | A36 |
| **206** | F | C1 | A21 | **499** | I | C2 | A6 | **792** | B | C2 | A36 |
| **207** | G | C1 | A21 | **500** | J | C2 | A6 | **793** | C | C2 | A36 |
| **208** | H | C1 | A21 | **501** | A | C2 | A7 | **794** | D | C2 | A36 |
| **209** | I | C1 | A21 | **502** | B | C2 | A7 | **795** | E | C2 | A36 |
| **210** | J | C1 | A21 | **503** | C | C2 | A7 | **796** | F | C2 | A36 |
| **211** | A | C1 | A22 | **504** | D | C2 | A7 | **797** | G | C2 | A36 |
| **212** | B | C1 | A22 | **505** | E | C2 | A7 | **798** | H | C2 | A36 |
| **213** | C | C1 | A22 | **506** | F | C2 | A7 | **799** | I | C2 | A36 |
| **214** | D | C1 | A22 | **507** | G | C2 | A7 | **800** | J | C2 | A36 |
| **215** | E | C1 | A22 | **508** | H | C2 | A7 | **801** | A | C2 | A37 |
| **216** | F | C1 | A22 | **509** | I | C2 | A7 | **802** | B | C2 | A37 |
| **217** | G | C1 | A22 | **510** | J | C2 | A7 | **803** | C | C2 | A37 |
| **218** | H | C1 | A22 | **511** | A | C2 | A8 | **804** | D | C2 | A37 |
| **219** | I | C1 | A22 | **512** | B | C2 | A8 | **805** | E | C2 | A37 |
| **220** | J | C1 | A22 | **513** | C | C2 | A8 | **806** | F | C2 | A37 |
| **221** | A | C1 | A23 | **514** | D | C2 | A8 | **807** | G | C2 | A37 |
| **222** | B | C1 | A23 | **515** | E | C2 | A8 | **808** | H | C2 | A37 |
| **223** | C | C1 | A23 | **516** | F | C2 | A8 | **809** | I | C2 | A37 |
| **224** | D | C1 | A23 | **517** | G | C2 | A8 | **810** | J | C2 | A37 |
| **225** | E | C1 | A23 | **518** | H | C2 | A8 | **811** | A | C2 | A38 |
| **226** | F | C1 | A23 | **519** | I | C2 | A8 | **812** | B | C2 | A38 |
| **227** | G | C1 | A23 | **520** | J | C2 | A8 | **813** | C | C2 | A38 |
| **228** | H | C1 | A23 | **521** | A | C2 | A9 | **814** | D | C2 | A38 |
| **229** | I | C1 | A23 | **522** | B | C2 | A9 | **815** | E | C2 | A38 |
| **230** | J | C1 | A23 | **523** | C | C2 | A9 | **816** | F | C2 | A38 |
| **231** | A | C1 | A24 | **524** | D | C2 | A9 | **817** | G | C2 | A38 |
| **232** | B | C1 | A24 | **525** | E | C2 | A9 | **818** | H | C2 | A38 |
| **233** | C | C1 | A24 | **526** | F | C2 | A9 | **819** | I | C2 | A38 |
| **234** | D | C1 | A24 | **527** | G | C2 | A9 | **820** | J | C2 | A38 |
| **235** | E | C1 | A24 | **528** | H | C2 | A9 | **821** | A | C2 | A39 |
| **236** | F | C1 | A24 | **529** | I | C2 | A9 | **822** | B | C2 | A39 |
| **237** | G | C1 | A24 | **530** | J | C2 | A9 | **823** | C | C2 | A39 |
| **238** | H | C1 | A24 | **531** | A | C2 | A10 | **824** | D | C2 | A39 |
| **239** | I | C1 | A24 | **532** | B | C2 | A10 | **825** | E | C2 | A39 |
| **240** | J | C1 | A24 | **533** | C | C2 | A10 | **826** | F | C2 | A39 |
| **241** | A | C1 | A25 | **534** | D | C2 | A10 | **827** | G | C2 | A39 |
| **242** | B | C1 | A25 | **535** | E | C2 | A10 | **828** | H | C2 | A39 |
| **243** | C | C1 | A25 | **536** | F | C2 | A10 | **829** | I | C2 | A39 |
| **244** | D | C1 | A25 | **537** | G | C2 | A10 | **830** | J | C2 | A39 |
| **245** | E | C1 | A25 | **538** | H | C2 | A10 | **831** | A | C2 | A40 |
| **246** | F | C1 | A25 | **539** | I | C2 | A10 | **832** | B | C2 | A40 |
| **247** | G | C1 | A25 | **540** | J | C2 | A10 | **833** | C | C2 | A40 |
| **248** | H | C1 | A25 | **541** | A | C2 | A11 | **834** | D | C2 | A40 |
| **249** | I | C1 | A25 | **542** | B | C2 | A11 | **835** | E | C2 | A40 |
| **250** | J | C1 | A25 | **543** | C | C2 | A11 | **836** | F | C2 | A40 |
| **251** | A | C1 | A26 | **544** | D | C2 | A11 | **837** | G | C2 | A40 |
| **252** | B | C1 | A26 | **545** | E | C2 | A11 | **838** | H | C2 | A40 |
| **253** | C | C1 | A26 | **546** | F | C2 | A11 | **839** | I | C2 | A40 |
| **254** | D | C1 | A26 | **547** | G | C2 | A11 | **840** | J | C2 | A40 |
| **255** | E | C1 | A26 | **548** | H | C2 | A11 | **841** | A | C2 | A41 |
| **256** | F | C1 | A26 | **549** | I | C2 | A11 | **842** | B | C2 | A41 |
| **257** | G | C1 | A26 | **550** | J | C2 | A11 | **843** | C | C2 | A41 |
| **258** | H | C1 | A26 | **551** | A | C2 | A12 | **844** | D | C2 | A41 |
| **259** | I | C1 | A26 | **552** | B | C2 | A12 | **845** | E | C2 | A41 |
| **260** | J | C1 | A26 | **553** | C | C2 | A12 | **846** | F | C2 | A41 |
| **261** | A | C1 | A27 | **554** | D | C2 | A12 | **847** | G | C2 | A41 |
| **262** | B | C1 | A27 | **555** | E | C2 | A12 | **848** | H | C2 | A41 |
| **263** | C | C1 | A27 | **556** | F | C2 | A12 | **849** | I | C2 | A41 |
| **264** | D | C1 | A27 | **557** | G | C2 | A12 | **850** | J | C2 | A41 |
| **265** | E | C1 | A27 | **558** | H | C2 | A12 | **851** | A | C2 | A42 |
| **266** | F | C1 | A27 | **559** | I | C2 | A12 | **852** | B | C2 | A42 |
| **267** | G | C1 | A27 | **560** | J | C2 | A12 | **853** | C | C2 | A42 |
| **268** | H | C1 | A27 | **561** | A | C2 | A13 | **854** | D | C2 | A42 |
| **269** | I | C1 | A27 | **562** | B | C2 | A13 | **855** | E | C2 | A42 |
| **270** | J | C1 | A27 | **563** | C | C2 | A13 | **856** | F | C2 | A42 |
| **271** | A | C1 | A28 | **564** | D | C2 | A13 | **857** | G | C2 | A42 |
| **272** | B | C1 | A28 | **565** | E | C2 | A13 | **858** | H | C2 | A42 |
| **273** | C | C1 | A28 | **566** | F | C2 | A13 | **859** | I | C2 | A42 |
| **274** | D | C1 | A28 | **567** | G | C2 | A13 | **860** | J | C2 | A42 |
| **275** | E | C1 | A28 | **568** | H | C2 | A13 | **861** | A | C2 | A43 |
| **276** | F | C1 | A28 | **569** | I | C2 | A13 | **862** | B | C2 | A43 |
| **277** | G | C1 | A28 | **570** | J | C2 | A13 | **863** | C | C2 | A43 |
| **278** | H | C1 | A28 | **571** | A | C2 | A14 | **864** | D | C2 | A43 |
| **279** | I | C1 | A28 | **572** | B | C2 | A14 | **865** | E | C2 | A43 |
| **280** | J | C1 | A28 | **573** | C | C2 | A14 | **866** | F | C2 | A43 |
| **281** | A | C1 | A29 | **574** | D | C2 | A14 | **867** | G | C2 | A43 |
| **282** | B | C1 | A29 | **575** | E | C2 | A14 | **868** | H | C2 | A43 |
| **283** | C | C1 | A29 | **576** | F | C2 | A14 | **869** | I | C2 | A43 |
| **284** | D | C1 | A29 | **577** | G | C2 | A14 | **870** | J | C2 | A43 |
| **285** | E | C1 | A29 | **578** | H | C2 | A14 | **871** | A | C2 | A44 |
| **286** | F | C1 | A29 | **579** | I | C2 | A14 | **872** | B | C2 | A44 |
| **287** | G | C1 | A29 | **580** | J | C2 | A14 | **873** | C | C2 | A44 |
| **288** | H | C1 | A29 | **581** | A | C2 | A15 | **874** | D | C2 | A44 |
| **289** | I | C1 | A29 | **582** | B | C2 | A15 | **875** | E | C2 | A44 |
| **290** | J | C1 | A29 | **583** | C | C2 | A15 | **876** | F | C2 | A44 |
| **291** | A | C1 | A30 | **584** | D | C2 | A15 | **877** | G | C2 | A44 |
| **292** | B | C1 | A30 | **585** | E | C2 | A15 | **878** | H | C2 | A44 |
| **293** | C | C1 | A30 | **586** | F | C2 | A15 | **879** | I | C2 | A44 |
| | | | | | | | | **880** | J | C2 | A44 |

Particularly preferred adjuvant compositions comprise A1, A4, A12 = A1+A3, A14 = A1+A9, A18 = A3+A4+A9, A21 = A1+A3+A9, A26 = A1+A24, A28 = A1+A9+A24, A29 = A1+A3+A24, A34 = A4+A24, A38 = A4+A3+A9 and A42 = A9+A23. These preferred adjuvant compositions can be combined with the mimotopes of the present invention to obtain a composition of the present invention.

The adjuvants mentioned in table A are well known in the art (see e.g. Reed SG, Trend Immunol 30(2008): 23-32).

According to a particularly preferred embodiment of the present invention the composition of the present invention comprises or consists of a combination of mimotopes, carriers and adjuvants selected from the group consisting of A-C1-A1, A-C1-A3, A-C1-A4/A5/A6, A-C1-A9, A-C1-A12, A-C1-A14, A-C1-A16, A-C1-A17, A-C1-A18, A-C1-A21, A-C1-A26, E-C1-A1, E-C1-A3, E-C1-A4/A5/A6, E-C1-A9, E-C1-A12, E-C1-A14, E-C1-A16, E-C1-A17, E-C1-A18, E-C1-A21, E-C1-A26, A-C2-A1, A-C2-A3, A-C2-A4/A5/A6, A-C2-A9, A-C2-A12, A-C2-A14, A-C2-A16, A-C2-A17, A-C2-A18, A-C2-A21, A-C2-A26, E-C2-A1, E-C2-A3, E-C2-A4/A5/A6, E-C2-A9, E-C2-A12, E-C2-A14, E-C2-A16, E-C2-A17, E-C2-A18, E-C2-A21 and E-C2-A26, preferably A-C1-A1, A-C1-A14, A-C1-A18,A-C1-A26, E-C1-A1, E-C1-A14, E-C1-A18, E-C1-A26, A-C2-A1, A-C2-A14, A-C2-A18,A-C2-A26, E-C2-A1, E-C2-A14, E-C2-A18 and E-C2-A26 whereby the variables are defined as in Table A (see above).

A further aspect of the present invention relates to a method for preventing and/or treating synucleinopathies as defined herein by administering to a subject in need thereof an appropriate amount of a composition as defined in the claims.

The term "preventing", as used herein, covers measures not only to prevent the occurrence of disease, such as risk factor reduction, but also to arrest its progress and reduce its consequences once established.

As used herein, the term "treatment" or grammatical equivalents encompasses the improvement and/or reversal of the symptoms of disease (e.g., neurodegenerative disease). A compound which causes an improvement in any parameter associated with disease when used in the screening methods of the instant invention may thereby be identified as a therapeutic compound. The term "treatment" refers to both therapeutic treatment and prophylactic or preventative measures. For example, those who may benefit from treatment with compositions and methods of the present invention include those already with a disease and/or disorder (e.g., neurodegenerative disease, lack of or loss of cognitive function) as well as those in which a disease and/or disorder is to be prevented (e.g., using a prophylactic treatment of the present invention).

The present invention is further defined in the following embodiments:
1. Composition comprising at least one mimotope of an epitope of alpha-synuclein for use in a method for preventing and/or treating ß-amyloidoses including Alzheimer's disease, wherein said at least one mimotope is coupled or fused to a pharmaceutically acceptable carrier protein selected from the group consisting of a non-toxic diphtheria toxin mutant, keyhole limpet hemocyanin (KLH), diphtheria toxin (DT), tetanus toxid (TT) and Haemophilus influenzae protein D (protein D).
2. Composition according to embodiment 1, wherein the non-toxic diphtheria toxin mutant is selected from the group consisting of CRM 197, CRM 176, CRM 228, CRM 45, CRM 9, CRM 102, CRM 103 and CRM 107, in particular CRM 197.
3. Composition according to embodiment 1 or 2, wherein the at least one mimotope is formulated for subcutaneous, intradermal, transdermal or intramuscular administration.
4. Composition according to any one of embodiments 1 to 3, wherein the at least one mimotope is formulated with at least one adjuvant.
5. Composition according to embodiment 4, wherein at least one adjuvant is capable to stimulate the innate immune system.
6. Composition according to embodiment 5, wherein at least one adjuvant capable to stimulate the innate immune system comprises or consists of a Toll-like receptor (TLR) agonist, preferably a TLR1, TLR2, TLR3, TLR4, TLR5, TLR7, TLR8 or TLR9 agonist, particularly preferred a TLR4 agonist.
7. Composition according to embodiment 6, wherein the TLR agonist is selected from the group consisting of monophosphoryl lipid A (MPL), 3-de-O-acylated monophosphoryl lipid A (3D-MPL), poly I:C, GLA, flagellin, R848, imiquimod and CpG.
8. Composition according to any one of embodiments 4 to 7, wherein the at least one adjuvant comprises or consists of a saponin, preferably QS21, a water in oil emulsion and a liposome.
9. Composition according to embodiment 4, wherein the at least one adjuvant is selected from the group consisting of MF59, AS01, AS02, AS03, AS04, aluminium hydroxide and aluminium phosphate.
10. Composition according to any one of embodiments 1 to 9, wherein the epitope comprises the amino acid sequence KNEEGAP or DMPVDPDN.
11. Composition according to any one of embodiments 1 to 10, wherein the at least one mimotope comprises the amino acid sequence

   (X₁)ₙX₂X₃X₄X₅GX₆P(X₇)ₘ (Formula I),

   wherein
   X₁ is any amino acid residue,
   X₂ is an amino acid residue selected from the group consisting of lysine (K), arginine (R), alanine (A) and histidine (H),
   X₃ is an amino acid residue selected from the group consisting of asparagine (N), glutamine (Q), serine (S), glycine (G) and alanine (A), preferably asparagine (N), serine (S), glycine (G) and alanine (A),
   X₄ is an amino acid residue selected from the group consisting of glutamic acid (E), aspartic acid (D) and alanine (A), X₅ is an amino acid residue selected from the group consisting of glutamic acid (E) and aspartic acid (D),
   X₆ is an amino acid residue selected from the group consisting of alanine (A) and tyrosine (Y),
   X₇ is any amino acid residue,
   n and m, independently, are 0 or an integer of more than 0, wherein the amino acid sequence according to Formula I is not identical with, or does not comprise the 7-mer polypeptide fragment of alpha-synuclein having the amino acid sequence KNEEGAP, and wherein
   the at least one mimotope comprising the amino acid sequence according to Formula I has a binding capacity to an antibody which is specific for an epitope of alpha-synuclein comprising the amino acid sequence KNEEGAP.
12. Composition according to embodiment 11, wherein X₂ is an amino acid residue selected from the group consisting of lysine (K) and arginine (R) and/or X₆ is alanine (A).
13. Composition according to embodiment 11 or 12, wherein the mimotope comprises an amino acid sequence selected from the group consisting of (X₁)ₙKNDEGAP(X₇)ₘ, (X₁)ₙANEEGAP(X₇)ₘ, (X₁)ₙKAEEGAP(X₇)ₘ, (X₁)ₙKNAEGAP(X₇)ₘ, (X₁)ₙRNEEGAP(X₇)ₘ, (X₁)ₙHNEEGAP(X₇)ₘ, (X₁)ₙKNEDGAP(X₇)ₘ, (X₁)ₙKQEEGAP(X₇)ₘ, (X₁)ₙKSEEGAP(X₇)ₘ, (X₁)ₙKNDDGAP(X₇)ₘ, (X₁)ₙRNDEGAP(X₇)ₘ, (X₁)ₙRNEDGAP(X₇)ₘ, (X₁)ₙRQEEGAP(X₇)ₘ, (X₁)ₙRSEEGAP(X₇)ₘ, (X₁)ₙANDEGAP(X₇)ₘ, (X₁)ₙANEDGAP(X₇)ₘ, (X₁)ₙHSEEGAP(X₇)ₘ, (X₁)ₙASEEGAP(X₇)ₘ, (X₁)ₙHNEDGAP(X₇)ₘ, (X₁)ₙHNDEGAP(X₇)ₘ, (X₁)ₙRNAEGAP(X₇)ₘ, (X₁)ₙHNAEGAP(X₇)ₘ, (X₁)ₙKSAEGAP(X₇)ₘ, (X₁)ₙKSDEGAP(X₇)ₘ, (X₁)ₙKSEDGAP(X₇)ₘ, (X₁)ₙRQDEGAP(X₇)ₘ, (X₁)ₙRQEDGAP(X₇)ₘ, (X₁)ₙHSAEGAP(X₇)ₘ, (X₁)ₙRSAEGAP(X₇)ₘ, (X₁)ₙRSDEGAP(X₇)ₘ, (X₁)ₙRSEDGAP(X₇)ₘ, (X₁)ₙHSDEGAP(X₇)ₘ, (X₁)ₙHSEDGAP(X₇)ₘ, (X₁)ₙRQDDGAP(X₇)ₘ, preferably (X₁)ₙKNDEGAP(X₂)ₘ, (X₁)ₙRNEEGAP (X₂)ₘ, (X₁)ₙRNDEGAP(X₂)ₘ, (X₁)ₙKNAEGAP(X₂)ₘ, (X₁)ₙKSDEGAP(X₂) ₘ, (X₁)ₙRNAEGAP(X₂)ₘ or (X₁)ₙRSEEGAP(X₂)ₘ.
14. Composition according to any one of embodiments 1 to 13 comprising at least one mimotope comprising an amino acid sequence selected from the group consisting of (X₁)ₙQASFAME(X₇)ₘ, (X₁)ₙTASWKGE(X₇)ₘ, (X₁)ₙQASSKLD(X₇)ₘ, (X₁)ₙTPAWKGE(X₇)ₘ, (X₁)ₙTPSWAGE(X₇)_{m,} (X₁)ₙTPSWKGE(X₇)ₘ,
   wherein
   X₁ is any amino acid residue,
   X₇ is any amino acid residue,
   n and m, independently, are 0 or an integer of more than 0,
   said at least one mimotope having a binding capacity to an antibody which is specific for an epitope of alpha-synuclein comprising the amino acid sequence KNEEGAP
   for use in preventing and/or treating synucleinopathies.
15. Composition according to any one of embodiments 1 to 14, wherein the at least one mimotope comprises the amino acid sequence

   (X_{1'})_{n'}X_{2'}X_{3'}PVX_{4'}X_{5'}X_{6'}(X_{7'})_{m'} (Formula II),

   wherein
   X_{1'} is any amino acid residue,
   X_{2'} is an amino acid residue selected from the group consisting of aspartic acid (D) and glutamic acid (E), X_{3'} is any amino acid residue,
   X_{4'} is any amino acid residue,
   X_{5'} is an amino acid residue selected from the group consisting of proline (P) and alanine (A),
   X_{6'} is an amino acid residue selected from the group consisting of aspartic acid (D) and glutamic acid (E), X_{7'} is any amino acid residue,
   n' and m', independently, are 0 or an integer of more than 0,
   wherein the amino acid sequence according to Formula II is not identical with, or does not comprise the 8-mer polypeptide fragment of alpha-synuclein having the amino acid sequence DMPVDPDN, and wherein
   the at least one mimotope comprising the amino acid sequence according to Formula II has a binding capacity to an antibody which is specific for an epitope of alpha-synuclein comprising the amino acid sequence DMPVDPDN.
16. Composition according to embodiment 15, wherein X_{3'} is an amino acid residue selected from the group consisting of glutamine (Q), serine (S), threonine (T), arginine (R), asparagine (N), valine (V), histidine (H), methionine (M), tyrosine (Y), alanine (A) and leucin (L).
17. Composition according to embodiment 15 or 16, wherein X_{4'} is an amino acid residue selected from the group consisting of glutamine (Q), tryptophane (W), threonine (T), arginine (R), aspartic acid(D), isoleucin (I), valine (V), histidine (H), proline (P), tyrosine (Y), alanine (A), serine (S) and leucin (L).
18. Composition according to any one of embodiments 15 to 17, wherein the mimotope has an amino acid sequence selected from the group consisting of (C)DQPVLPD, (C)DMPVLPD, (C)DSPVLPD, (C)DSPVWAE, (C)DTPVLAE, (C)DQPVLPDN, (C)DMPVLPDN, (C)DSPVLPDN, (C)DQPVTAEN, (C)DSPVWAEN, (C)DTPVLAEN, (C)HDRPVTPD, (C)DRPVTPD, (C)DVPVLPD, (C)DTPVYPD, (C)DTPVIPD, (C)HDRPVTPDN, (C)DRPVTPDN, (C)DNPVHPEN, (C)DVPVLPDN, (C)DTPVYPDN, (C)DTPVIPDN, (C)DQPVLPDG, (C)DMPVLPDG, (C)DSPVLPDG, (C)DSPVWAEG, (C)DRPVAPEG, (C)DHPVHPDS, (C)DMPVSPDR, (C)DSPVPPDD, (C)DQPVYPDI, (C)DRPVYPDI, (C)DHPVTPDR, (C)EYPVYPES, (C)DTPVLPDS, (C)DMPVTPDT, (C)DAPVTPDT, (C)DSPVVPDN, (C)DLPVTPDR, (C)DSPVHPDT, (C)DAPVRPDS, (C)DMPVWPDG, (C)DAPVYPDG, (C)DRPVQPDR, (C)YDRPVQPDR, (C)DMPVDPEN, (C)DMPVDADN, DQPVLPD(C), DMPVLPD(C), (C)EMPVDPDN and (C)DNPVHPE.
19. Composition according to any one of embodiments 11 to 17, characterised in that n' and/or m' are 1 and X_{1'} and/or X_{7'} are cysteine (C).
20. Composition according to any one of embodiments 11 to 19, wherein the mimotope comprises 7 to 30, preferably 7 to 20, more preferably 7 to 16, most preferably 8 or 9, amino acid residues.
21. Composition according to any one of embodiments 1 to 20, wherein the at least one mimotope is selected from the group of DQPVLPD, DSPVLPD, DVPVLPD, DSPVLPDG, YDRPVQPDR, DHPVHPDS, DAPVRPDS, KNDEGAP, KQEEGAP and KSEEGAP, in particular DQPVLPD and YDRPVQPDR
22. Composition according to any one of embodiments 1 to 21 comprising a combination of at least one mimotope and carrier and/or adjuvant as defined in Table A, preferably A-C1-A1, A-C1-A14, A-C1-A18,A-C1-A26, E-C1-A1, E-C1-A14, E-C1-A18, E-C1-A26, A-C2-A1, A-C2-A14, A-C2-A18,A-C2-A26, E-C2-A1, E-C2-A14, E-C2-A18 and E-C2-A26.

The present invention is further illustrated by the following examples, however, without being restricted thereto.

### EXAMPLES

### Material and Methods:

### In vivo characterisation of mimotope-vaccine candidates: Conjugate production:

Mimotope peptides were coupled to the carrier CRM-197 or KLH by using the heterobifunctional crosslinking agent GMBS. Briefly, CRM-197/KLH was mixed with an excess of GMBS at room temperature to allow for activation, followed by removal of excess GMBS by dialysis. Excess mimotope peptide was then added to the activated carrier. The mimotope CRM-197/KLH conjugate was used for vaccine formulation.

Vaccines were formulated with different adjuvants and applied to animals. Identical amounts of conjugated mimotope peptide(s) were injected per mouse when the CRM-197/KLH vaccines were compared to other vaccines or when different adjuvants were compared.

### Animal experiments:

Female BALB/c mice, 6 mice per group, were immunized with mimotope-CRM-197/KLH conjugates using different adjuvants. Control groups were immunized with CRM-197/KLH plus respective adjuvants and/or PBS and/or adjuvants alone.

Animals were vaccinated 3-6 times in regular intervals (2-4 week interval) and plasma samples were taken regularly as well (one day before vaccination).

### Example 1: Effect of mimotope-CRM197 conjugates using different adjuvant systems:Immunogenicity

In several parallel experiments, female BALB/c mice are immunized repeatedly with identical amounts of AFFITOPE peptides (the mimotopes disclosed herein), comprising preferably a C or N-terminal cysteine residue, coupled to CRM-197 (e.g. 1-10µg peptide per immunisation). Different formulations using the same AFFITOPE conjugate are compared to suitable control groups (e.g.: PBS alone or adjuvant alone or CRM197 plus adjuvant)

The following peptide-conjugates or combinations of conjugates are used:
● DQPVLPD coupled to CRM197
● DSPVLPD coupled to CRM197
● DVPVLPD coupled to CRM197
● DSPVLPDG coupled to CRM197
● YDRPVQPDR coupled to CRM197
● DHPVHPDS coupled to CRM197
● DAPVRPDS coupled to CRM197
● KNDEGAP coupled to CRM197
● KQEEGAP coupled to CRM197
● KSEEGAP coupled to CRM197

### Adjuvants used in this example are:

Aluminium hydroxide, squalene-based, oil in water emulsion(s), Saponin containing adjuvants; Adjuvants are also containing TLR agonists (e.g.: TLR4)

The in vitro ELISA assay to determine the antibody titer following immunisation is performed with plasma of single mice (see method description below). The plasma samples obtained in this example are also assessed for the presence of peptide specific IgG subtypes induced by the conjugate vaccine.

### Peptide ELISA:

In order to perform ELISAs for detecting the immune responses in vaccinated animals, peripheral blood was drawn from mice using heparin as anticoagulant and plasma was prepared from these samples. The diluted plasma was then used for ELISA analysis. For this purpose, the wells of the ELISA plates (Nunc Maxisorb) were coated with peptide-BSA conjugates. Subsequently, diluted plasma was added and the detection of peptide specific antibodies was performed with biotinylated anti-mouse IgG (Southern Biotech) and subsequent colour reaction using Streptavidin-POD (Roche) and ABTS. For IgG subtype analyses, specific biotinylated anti murine IgG1, IgG2a, IgG2b and IgG3 antibodies (Southern Biotech) were used.

### Example 2: Effect of mimotope-KLH conjugates using different adjuvant systems:Immunogenicity

In several parallel experiments, female BALB/c mice are immunized repeatedly with identical amounts of mimotope peptides coupled to KLH (e.g. 1-30µg peptide per immunisation). Different formulations using the same mimotope conjugate are compared to suitable control groups (e.g.: PBS alone or adjuvant alone or KLH plus adjuvant)

The following peptide-conjugates or combinations of conjugates are used:
● DQPVLPD coupled to KLH
● DSPVLPD coupled to KLH
● DVPVLPD coupled to KLH
● DSPVLPDG coupled to KLH
● YDRPVQPDR coupled to KLH
● DHPVHPDS coupled to KLH
● DAPVRPDS coupled to KLH
● KNDEGAP coupled to KLH
● KQEEGAP coupled to KLH
● KSEEGAP coupled to KLH

### Adjuvants used in this example are:

Aluminium hydroxide, squalene-based, oil in water emulsion(s), Saponin containing adjuvants (with liposome); Adjuvants are also containing TLR agonists (e.g.: TLR4, TLR9, TLR7 or TLR2/4)

The in vitro ELISA assay to determine the antibody titer following immunisation is performed with plasma of single mice (see method description as in example 1). The plasma samples obtained in this experiment are also assessed for the presence of peptide specific IgG subtypes induced by the conjugate vaccine.

### Example 3: Effect of mimotope-CRM197 conjugates using different adjuvant systems: effect on peripheral monocyte/macrophage

In order to analyse whether mimotope-CRM197 adjuvanted with the different adjuvants described before, is able to change the cytokine milieu and thus influence peripheral monocyte/macrophage activation, the levels of Cytokines/Chemokines known to activate monocytes/macrophages or indicating monocyte/macrophage activation (e.g. CCL2/MCP1 etc.) were determined. Cytokine/Chemokine levels are determined in plasma from treated animals 3 hours after injection of the different vaccines.

### Cytokine determination:

To determine the concentration of cytokines in the circulation of vaccinated animals, blood was collected from animals 2-4 hours after injection of vaccines. Subsequently, plasma was prepared from blood samples and cytokine concentration in individual samples was defined using the FlowCytomix bead array system (eBioscience) and flow cytometric analysis.

### Example 4: Effect of mimotope-KLH conjugates using different adjuvant systems: effect on peripheral monocyte/macrophage

In order to analyse whether mimotope-CRM197 adjuvanted with the different adjuvants described before, is able to change the cytokine milieu and thus influence peripheral monocyte/macrophage activation, the levels of Cytokines/Chemokines known to activate monocytes/macrophages or indicating monocyte/macrophage activation (e.g. CCL2/MCP1 etc.) were determined. Cytokine/Chemokine levels are determined in plasma from treated animals 3 hours after injection of the different vaccines (for details see method in example 3).

### Example 5: Effect of immunotherapy on monocytes and monocytic alpha synuclein uptake

The ability of the novel vaccine formulations to alter peripheral CD11b+ monocyte numbers as well as to change monocytic alpha Synuclein uptake in vivo is also assessed.

As described previously, monocytes are considered the peripheral blood precursor cells of brain microglia (Rezaie, P., et al 1999. Dev. Brain Res. 115:71-81; Mildner et al Nat Neurosci. 2007 Dec;10(12):1544-53). Markers such as CD11b and Ly6C are immunologicals markers that are present on such peripheral blood monocytes and persist when these cells are infiltrating the brain (Mildner et al., 2007, Lebson L, et al. J Neurosci. 2010 Jul 21;30(29):9651-8).

To investigate whether TLR agonist containing adjuvants or components thereof are contributing to changing the number of monocytes in the peripheral blood, a comparative analysis of the conjugate-formulations mentioned before is performed.

This result is again demonstrating a synergistic effect of mimotope-vaccine induced immune responses (antibodies) with a TLR agonists used in the adjuvant.

### Flow cytometry analysis:

Peripheral blood was drawn from mice with lithium-heparin as anticoagulant, 24-Hour after last injection of the vaccines and antibodies, respectively. Red blood cell lysis was performed twice on pooled blood with Ammonium Chloride-based Buffer (Sigma, Steinheim, Germany) and cells were counted. Remaining peripheral blood cells were incubated with Rat anti- Mouse CD16/CD32 (BD Fc Block™ by BD Biosciences) and cells were further incubated with a combination of directly conjugated antibodies as described by Mildner et al., 2007 or similar antibodies: PE-conjugated Hamster anti-Mouse CD3, Rat anti-Mouse CD45R/B220, Rat anti-Mouse Ly-6G, Mouse anti-Mouse NK1.1; APC-conjugated Rat anti-Mouse CD11b; PE-Cy7-conjugated Hamster anti-Mouse CD11c, FITC- Rat Anti-Mouse Ly-6C and a suitable Rat anti-Mouse CD62L.

Samples were acquired on a flow cytometer (BD FACSCanto II) and data were analyzed with the FACSDiva software (BD Biosciences) including the automated compensation protocol for the used fluorescence channels.

Monocytes were identified by their Forward/Side scatter properties and gated as CD3-/CD45R/B220-/Ly-6G-/NK1.1-(Lineage-)/CD11b+ cells. CD11b+ monocyte frequency was reported as a percentage of the total cells (excluding debris).

### Alpha Synuclein uptake assay:

To examine the function of monocytes in the peripheral blood, the capacity of those monocytes to uptake recombinant human alpha synuclein was examined. In order to measure the phagocytic activity, fluorescent recombinant human alpha-synuclein(1-140; HiLyte Fluor™488 labeled, Anaspec Inc.) was used.

For that analysis mice were injected with HiLyte Fluor™488 labeled alpha-synuclein and blood was withdrawn 2h after injection. Samples for alpha synuclein uptake determination were acquired on a flow cytometer (BD FACSCanto II) and data analyzed with the FACSDiva software (BD Biosciences).

Monocytes were identified by their Side/Forward scatter properties, excluding debris and gated as CD3-/CD45R/B220-/Ly-6G-/NK1.1-(Lineage-)/CD11b+ cells. Alpha synuclein uptake was assessed by reporting the percentage and Mean Fluorescence Intensity of HiLyte fluor™ 488 alpha synuclein positive cells among gated monocytes.

## Claims

1. Composition comprising at least one mimotope of an epitope of alpha-synuclein for use in a method for preventing and/or treating ß-amyloidoses including Alzheimer's disease, wherein said at least one mimotope is coupled or fused to a pharmaceutically acceptable carrier protein selected from the group consisting of a non-toxic diphtheria toxin mutant, keyhole limpet hemocyanin (KLH), diphtheria toxin (DT), tetanus toxid (TT) and Haemophilus influenzae protein D (protein D).

2. Composition according to claim 1, wherein the non-toxic diphtheria toxin mutant is selected from the group consisting of CRM 197, CRM 176, CRM 228, CRM 45, CRM 9, CRM 102, CRM 103 and CRM 107, in particular CRM 197.

3. Composition according to claim 1 or 2, wherein the at least one mimotope is formulated with at least one adjuvant.

4. Composition according to claim 3, wherein at least one adjuvant is capable to stimulate the innate immune system.

5. Composition according to claim 4, wherein wherein at least one adjuvant capable to stimulate the innate immune system comprises or consists of a Toll-like receptor (TLR) agonist, preferably a TLR1, TLR2, TLR3, TLR4, TLR5, TLR7, TLR8 or TLR9 agonist, particularly preferred a TLR4 agonist.

6. Composition according to claim 5, wherein the TLR agonist is selected from the group consisting of monophosphoryl lipid A (MPL), 3-de-O-acylated monophosphoryl lipid A (3D-MPL), poly I:C, GLA, flagellin, R848, imiquimod and CpG.

7. Composition according to any one of claims 3 to 6, wherein the at least one adjuvant comprises or consists of a saponin, preferably QS21, a water in oil emulsion and a liposome.

8. Composition according to claim 3, wherein the at least one adjuvant is selected from the group consisting of MF59, AS01, AS02, AS03, AS04, aluminium hydroxide and aluminium phosphate.

9. Composition according to any one of claims 1 to 8, wherein the epitope comprises the amino acid sequence KNEEGAP or DMPVDPDN.

10. Composition according to any one of claims 1 to 9, wherein the at least one mimotope comprises the amino acid sequence
(X₁)ₙX₂X₃X₄X₅GX₆P(X₇)ₘ (Formula I),
wherein
X₁ is any amino acid residue,
X₂ is an amino acid residue selected from the group consisting of lysine (K), arginine (R), alanine (A) and histidine (H),
X₃ is an amino acid residue selected from the group consisting of asparagine (N), glutamine (Q), serine (S), glycine (G) and alanine (A), preferably asparagine (N), serine (S), glycine (G) and alanine (A),
X₄ is an amino acid residue selected from the group consisting of glutamic acid (E), aspartic acid (D) and alanine (A), X₅ is an amino acid residue selected from the group consisting of glutamic acid (E) and aspartic acid (D),
X₆ is an amino acid residue selected from the group consisting of alanine (A) and tyrosine (Y),
X₇ is any amino acid residue,
n and m, independently, are 0 or an integer of more than 0, wherein the amino acid sequence according to Formula I is not identical with, or does not comprise the 7-mer polypeptide fragment of alpha-synuclein having the amino acid sequence KNEEGAP, and wherein
the at least one mimotope comprising the amino acid sequence according to Formula I has a binding capacity to an antibody which is specific for an epitope of alpha-synuclein comprising the amino acid sequence KNEEGAP.

11. Composition according to claim 10, wherein the mimotope comprises an amino acid sequence selected from the group consisting of (X₁)ₙKNDEGAP(X₇)ₘ, (X₁)ₙANEEGAP(X₇)ₘ, (X₁)ₙKAEEGAP(X₇)ₘ, (X₁)ₙKNAEGAP(X₇)ₘ, (X₁)ₙRNEEGAP(X₇)ₘ, (X₁)ₙHNEEGAP(X₇)ₘ, (X₁)ₙKNEDGAP(X₇)ₘ, (X₁)ₙKQEEGAP(X₇)ₘ, (X₁)ₙKSEEGAP(X₇)ₘ, (X₁)ₙKNDDGAP(X₇)ₘ, (X₁)ₙRNDEGAP(X₇)ₘ, (X₁)ₙRNEDGAP(X₇)ₘ, (X₁)ₙRQEEGAP(X₇)ₘ, (X₁)ₙRSEEGAP(X₇)ₘ, (X₁)ₙANDEGAP(X₇)ₘ, (X₁)ₙANEDGAP(X₇)ₘ, (X₁)ₙHSEEGAP(X₇)ₘ, (X₁)ₙASEEGAP(X₇)ₘ, (X₁)ₙHNEDGAP(X₇)ₘ, (X₁)ₙHNDEGAP(X₇)ₘ, (X₁)ₙRNAEGAP(X₇)ₘ, (X₁)ₙHNAEGAP(X₇)ₘ, (X₁)ₙKSAEGAP(X₇)ₘ, (X₁)ₙKSDEGAP(X₇)ₘ, (X₁)ₙKSEDGAP(X₇)ₘ, (X₁)ₙRQDEGAP(X₇)ₘ, (X₁)ₙRQEDGAP(X₇)ₘ, (X₁)ₙHSAEGAP(X₇)ₘ, (X₁)ₙRSAEGAP(X₇)ₘ, (X₁)ₙRSDEGAP(X₇)ₘ, (X₁)ₙRSEDGAP(X₇)ₘ, (X₁)ₙHSDEGAP(X₇)ₘ, (X₁)ₙHSEDGAP(X₇)ₘ, (X₁)ₙRQDDGAP(X₇)ₘ, preferably (X₁)ₙKNDEGAP(X₂)ₘ, (X₁)ₙRNEEGAP(X₂)ₘ, (X₁)ₙRNDEGAP(X₂)ₘ, (X₁)ₙKNAEGAP(X₂)ₘ, (X₁)KSDEGAP(X₂)ₘ, (X₁)ₙRNAEGAP(X₂) or (X₁)ₙRSEEGAP(X₂)ₘ.

12. Composition according to any one of claims 1 to 11 comprising at least one mimotope comprising an amino acid sequence selected from the group consisting of (X₁)ₙQASFAME(X₇)ₘ, (X₁)ₙTASWKGE(X₇)ₘ, (X₁)ₙQASSKLD(X₇)ₘ, (X₁)ₙTPAWKGE(X₇)ₘ, (X₁)ₙTPSWAGE(X₇)ₘ, (X₁) ₙTPSWKGE(X₇)ₘ,
wherein
X₁ is any amino acid residue,
X₇ is any amino acid residue,
n and m, independently, are 0 or an integer of more than 0, said at least one mimotope having a binding capacity to an antibody which is specific for an epitope of alpha-synuclein comprising the amino acid sequence KNEEGAP.

13. Composition according to any one of claims 1 to 12, wherein the at least one mimotope comprises the amino acid sequence
(X_{1'})_{n'}X_{2'}X_{3'}PVX_{4'}X_{5'}X_{6'}(X_{7'})_{m'} (Formula II),
wherein
X_{1'} is any amino acid residue,
X_{2'} is an amino acid residue selected from the group consisting of aspartic acid (D) and glutamic acid (E),
X_{3'} is any amino acid residue,
X_{4'} is any amino acid residue,
X_{5'} is an amino acid residue selected from the group consisting of proline (P) and alanine (A),
X_{6'} is an amino acid residue selected from the group consisting of aspartic acid (D) and glutamic acid (E),
X_{7'} is any amino acid residue, n' and m', independently, are 0 or an integer of more than 0,
wherein the amino acid sequence according to Formula II is not identical with, or does not comprise the 8-mer polypeptide fragment of alpha-synuclein having the amino acid sequence DMPVDPDN, and wherein
the at least one mimotope comprising the amino acid sequence according to Formula II has a binding capacity to an antibody which is specific for an epitope of alpha-synuclein comprising the amino acid sequence DMPVDPDN.

14. Composition according to claim 3, wherein the mimotope has an amino acid sequence selected from the group consisting of (C)DQPVLPD, (C)DMPVLPD, (C)DSPVLPD, (C)DSPVWAE, (C)DTPVLAE, (C)DQPVLPDN, (C)DMPVLPDN, (C)DSPVLPDN, (C)DQPVTAEN, (C)DSPVWAEN, (C)DTPVLAEN, (C)HDRPVTPD, (C)DRPVTPD, (C)DVPVLPD, (C)DTPVYPD, (C)DTPVIPD, (C)HDRPVTPDN, (C)DRPVTPDN, (C)DNPVHPEN, (C)DVPVLPDN, (C)DTPVYPDN, (C)DTPVIPDN, (C)DQPVLPDG, (C)DMPVLPDG, (C)DSPVLPDG, (C)DSPVWAEG, (C)DRPVAPEG, (C)DHPVHPDS, (C)DMPVSPDR, (C)DSPVPPDD, (C)DQPVYPDI, (C)DRPVYPDI, (C)DHPVTPDR, (C)EYPVYPES, (C)DTPVLPDS, (C)DMPVTPDT, (C)DAPVTPDT, (C)DSPVVPDN, (C)DLPVTPDR, (C)DSPVHPDT, (C)DAPVRPDS, (C)DMPVWPDG, (C)DAPVYPDG, (C)DRPVQPDR, (C)YDRPVQPDR, (C)DMPVDPEN, (C)DMPVDADN, DQPVLPD(C), DMPVLPD(C), (C)EMPVDPDN and (C)DNPVHPE.

15. Composition according to any one of claims 10 to 14, **characterised in that** n' and/or m' are 1 and X_{1'} and/or X_{7'} are cysteine (C).

16. Composition according to any one of claims 1 to 15, wherein the at least one mimotope is selected from the group of DQPVLPD, DSPVLPD, DVPVLPD, DSPVLPDG, YDRPVQPDR, DHPVHPDS, DAPVRPDS, KNDEGAP, KQEEGAP and KSEEGAP, in particular DQPVLPD and YDRPVQPDR.
